(19) Europäisches Patentamt
European Patent Office
Office européen des brevets

(11) EP 1 855 646 B1

(12) EUROPEAN PATENT SPECIFICATION

(45) Date of publication and mention of the grant of the patent: **11.11.2009 Bulletin 2009/46**

(21) Application number: **06719386.2**

(22) Date of filing: **25.01.2006**

(51) Int Cl.:
***A61K 8/46*** *(2006.01)* ***A61K 8/27*** *(2006.01)*
***A61K 8/49*** *(2006.01)* ***A61Q 5/00*** *(2006.01)*

(86) International application number: **PCT/US2006/002499**

(87) International publication number: **WO 2006/083631 (10.08.2006 Gazette 2006/32)**

(54) **DIIODOMETHYL-P-TOLYLSULFONE AS A PARTICULATE DISPERSION IN A LIQUID SOLVENT**

DIIODOMETHYL-P-TOLYLSULFON ALS TEILCHENDISPERSION IN EINEM FLÜSSIGEN LÖSUNGSMITTEL

DIIODOMETHYL-P-TOLYLSULFONE EN TANT QUE DISPERSION PARTICULAIRE DANS UN SOLVANT LIQUIDE

(84) Designated Contracting States:
**AT BE BG CH CY CZ DE DK EE ES FI FR GB GR HU IE IS IT LI LT LU LV MC NL PL PT RO SE SI SK TR**

(30) Priority: **28.01.2005 US 648164 P**

(43) Date of publication of application: **21.11.2007 Bulletin 2007/47**

(73) Proprietor: **The Procter and Gamble Company**
**Cincinnati, Ohio 45202 (US)**

(72) Inventors:
- **SCHWARTZ, James Robert**
  **West Chester,**
  **Ohio 45069 (US)**
- **WARNKE, David Thomas**
  **Cincinnati,**
  **Ohio 45202 (US)**
- **KAUFMAN, David Joseph**
  **Fairfield,**
  **Ohio 45014 (US)**

(74) Representative: **Kohol, Sonia et al**
**Procter & Gamble Technical Centres Limited**
**Patent Department**
**Rusham Park**
**Whitehall Lane**
**Egham**
**Surrey TW20 9NW (GB)**

(56) References cited:
**EP-A- 0 034 877** **WO-A1-2006/066204**
**US-A- 3 806 351** **US-A1- 2003 096 545**

- **DATABASE CA [Online] CHEMICAL ABSTRACTS SERVICE, COLUMBUS, OHIO, US; KIM, CHANG-DUK ET AL: "Hair cosmetic compositions" XP002398529 retrieved from STN Database accession no. 2004:150147 & KR 141 416 B1 (LG CHEMICAL CO., LTD., S. KOREA) 1 June 1998 (1998-06-01)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 855 646 B1

## Description

### Field

The present invention relates to a composition comprising an effective amount of diiodomethyl-p-tolylsulfone wherein the diiodomethyl-p-tolylsulfone is present as a particulate dispersion in a liquid solvent. More particularly, the present invention relates to compositions and methods of treating microbial and fungal infections on the skin or scalp. Even more particularly, the present invention relates to methods for the treatment of topical fungally-mediated conditions, especially dandruff.

### Background

Various anti-dandruff compositions are commercially available or otherwise known in the shampoo art. These compositions typically comprise detersive surfactants and particulate, crystalline anti-microbial agents dispersed and suspended throughout the composition. Anti-microbial agents used for this purpose include sulfur, selenium sulfide and polyvalent metal salts of pyridinethione. During the shampooing process, these antimicrobial agents deposit on the scalp to provide anti-dandruff activity. Soluble antidandruff agents, such as ketoconazole and octopirox, are also known in the art.

Diiodomethyl-p-tolylsulfone is an antimicrobial agent which is useful for the control of microbial degradation in a variety of end-use applications. It is EPA-registered for use in adhesives, paper coatings, plastics, tanned leather, caulks, metalworking fluids, textiles, coatings and wood preservation.

US2003/096545A1 discloses antimicrobial, sporicidal compositions useful in the treatment of bacterial and fungal spores. The compositions comprise dispersion of a iodine containing compound and pyrithione. Pyrithione may be in the form of sodium pyrithione, zinc pyrithione, copper pyrithione, or silver pyrithione. The iodine containing compound can be diiodomethyl-4-tolylsulfone.

KR-B-0 141 416 (Chang-Duk Kim et al.) relates to a cosmetic composition for hair, suitable for preventing and curing dandruff and itching of the head skin, said composition comprising diiodomethyl-p-tolylsulfone and piroctonolamine, and to a method for preparing said composition.

EP 0 034 877 A discloses room temperature vulcanizable (RTV) silicone rubber compositions which are mildew resistant. The compositions comprise a silanol endstopped diorganopolysiloxane, a cross-linking agent, a curing catalyst, and an effective amount of a fungicide. The fungicide can be diiodomethyl-p-tolylsulfone.

However, even though diiodomethyl-p-tolylsulfone is known as a preservation agent with desirable fungicidal activity, a great disadvantage of diiodomethyl-p-tolylsulfone is its extremely low water solubility of less than 5 ppm. Such characteristics present a challenge for the development of an efficacious composition because activity requires molecular bio-availability which is proportional to solubility.

Despite the options available, consumers still desire a shampoo that provides superior anti-dandruff efficacy versus currently marketed products; as such consumers have found that dandruff is still prevalent. Such a superior efficacy can be difficult to achieve.

### Summary

An embodiment of the present invention is directed to a composition comprising an effective amount of diiodomethyl-p-tolylsulfone wherein the diiodomethyl-p-tolylsulfone is present as a particulate dispersion in a liquid solvent.

An additional embodiment of the present invention is directed to a composition according to Claim 1 wherein at least about 30% retained anti-fungal activity is achieved as the diiodomethyl-p-tolylsulfone is present as a particulate dispersion in a liquid solvent relative to its activity in a solubilized state.

These and other features, aspects, and advantages of the present invention will become evident to those skilled in the art from a reading of the present disclosure.

### Detailed Description

While the specification concludes with claims, which particularly point out and distinctly claim the invention, it is believed the present invention will be better understood from the following description.

Biological active materials must interact with their target on a molecular level to exert their effect. Materials with very low solubility (especially those wherein a major proportion exists as a particulate dispersion in water) tend to have reduced activity vs. soluble versions. This places a high need on effective pharmacological delivery approaches to minimize the loss of activity due to the physical form of the active. The latter effect is desirable in that it has the benefit of increasing overall anti-microbial effectiveness.

Applicants have surprisingly found that diiodomethyl-p-tolylsulfone has been found to retain about 100% of its activity in a dispersed particle form vs. that dissolved in an effective solvent. Particulate materials are desirable so long as biological activity can be maintained, because physical delivery of the active is more efficient and the deposited material can serve as a reservoir of activity over time.

The present invention can comprise, consist of, or consist essentially of the essential elements and limitations of the invention described herein, as well any of the additional or optional ingredients, components, or limitations described herein.

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

The components and/or steps, including those, which may optionally be added, of the various embodiments of the present invention, are described in detail below.

All documents cited are, in relevant part, incorporated herein by reference; the citation of any document is not to be construed as an admission that it is prior art with respect to the present invention.

All ratios are weight ratios unless specifically stated otherwise.

All temperatures are in degrees Celsius, unless specifically stated otherwise.

Except as otherwise noted, all amounts including quantities, percentages, portions, and proportions, are understood to be modified by the word "about", and amounts are not intended to indicate significant digits.

Except as otherwise noted, the articles "a", "an", and "the" mean "one or more"

Herein, "comprising" means that other steps and other ingredients, which do not affect the end result, can be added. This term encompasses the terms "consisting of" and "consisting essentially of". The compositions and methods/processes of the present invention can comprise, consist of, and consist essentially of the essential elements and limitations of the invention described herein, as well as any of the additional or optional ingredients, components, steps, or limitations described herein.

Herein, "effective" means an amount of a subject active high enough to provide a significant positive modification of the condition to be treated. An effective amount of the subject active will vary with the particular condition being treated, the severity of the condition, the duration of the treatment, the nature of concurrent treatment, and like factors.

A. Diiodomethyl-p-tolylsulfone

B.

The compositions of the present invention comprise diiodomethyl-p-tolylsulfone. Diiodomethyl-p-tolylsulfone ($C_8H_8I_2O_2S$) is represented by formula I:

I

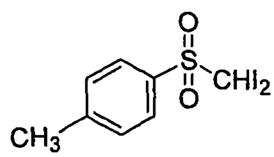

Diiodomethyl-p-tolylsulfone has the CAS Registry Number 20018-09-1. The CA Index name is listed as Benzene, 1-[(diiodomethyl)sulfonyl]-4-methyl-(9CI). Other names for diiodomethyl-p-tolylsulfone are as follows: Sulfone, diiodomethyl p-tolyl (8CI); 4-Tolyl diiodomethyl sulfone; A 9248; ABG 2000; AMICAL 48; AMICAL 50; AMICAL 81; AMICAL WP; DM 95; DP 1104; Diiodomethyl 4-tolyl sulfone; Diiodomethyl p-tolyl sulfone; Surfasept 74859; Ultra Fresh 95; Ultra Fresh DM 95; Ultrafresh UF 95; Yotoru D; p-Methylphenyl diiodomethyl sulfone; p-Tolyl diiodomethyl sulfone; p-[(Diiodomethyl)sulfonyl]toluol.

Diiodomethyl-p-tolylsulfone is commercially available under the tradename AMICAL™ (i.e. AMICAL 48, AMICAL Flowable, AMICAL WP) and is available from Dow. AMICAL™ preservatives are antimicrobial agents which are useful for the control of microbial degradation in a variety of end-use applications. They are EPA-registered for use in adhesives, paper coatings, plastics, tanned leather, caulks, metalworking fluids, textiles, coatings and wood preservation. The advantage of using the AMICAL™ preservative is it is extremely effective antifungal agent; FDA clearance in the U.S. for several indirect food contact applications; effective over a broad pH range (2-11); Non dermally-irritating. AMICAL™ preservatives are approved by the CFTA/INCI (Designation: Diiodomethyltolylsulfone) for use as a fungicide in cosmetic

preservation.

However, even though diiodomethyl-p-tolylsulfone is known as a preservation agent with fungicidal activity, a great disadvantage of diiodomethyl-p-tolylsulfone is its extremely low water solubility of less than 5 ppm.

Applicants have surprisingly found a manner by which the diiodomethyl-p-tolylsulfone is maintained as a particulate dispersion in a liquid solvent while maintaining anti-fungal efficacy.

Without being bound by theory, particulate dispersions are desirable because deposition efficiency is relatively high and a reservoir for active material is formed for benefits over time. The limitation is that particulates inherently have low solubility which limits the bio-availability of the active species. While diiodomethyl-p-tolylsulfone has very low aqueous solubility, it has been surprisingly found that bio-availability is not negatively effected and, therefore, represents an ideal material for application to topical anti-fungal products.

Preferred embodiments of the present invention include compositions comprising from about 0.001% to about 10% of diiodomethyl-p-tolylsulfone; more preferably from about 0.01% to about 5% diiodomethyl-p-tolylsulfone; more preferably from about 0.1% to about 3% diiodomethyl-p-tolylsulfone.

In the present invention, a liquid solvent is selected from the group consisting of water, water miscible co-solvents, liquids in which diiodomethyl-p-tolylsulfone remains predominantly insoluble wherein the diiodomethyl-p-tolylsulfone does not irreversibly aggregate (i.e. the diiodomethyl-p-tolylsulfone does not irreversibly aggregate), and mixtures thereof.

A stable dispersion may exist wherein it can be homogenously sampled immediately upon shaking, i.e. the stable dispersion may not irreversibly aggregate.

In an embodiment of the present invention, greater than about 70% of the diiodomethyl-p-tolylsulfone remains in the form of a particulate dispersion in a liquid solvent, preferably greater than about 80% of the diiodomethyl-p-tolylsulfone remains in the form of a particulate dispersion in a liquid solvent, and more preferably greater than about 90% of the diiodomethyl-p-tolylsulfone remains in the form of a particulate dispersion in a liquid solvent.

Particle Size of diiodomethyl-p-tolylsulfone

In an embodiment of the present invention, it is has been found that a smaller particle improves dispersion stability.

D(50) is the median particle size or the particle size which corresponds to 50% of the amount of particles are below this size In an embodiment of the present invention, the diiodomethyl-p-tolylsulfone may have a particle size distribution wherein 50% of the particles are less than about 30 microns. In a further embodiment of the present invention, the diiodomethyl-p-tolylsulfone may have a particle size distribution wherein 50% of the particles are less than about 15 microns. In yet a further embodiment of the present invention, the diiodomethyl-p-tolylsulfone may have a particle size distribution
wherein 50% of the particles are less than about 7.5 microns.

C. Topical Carrier

In a preferred embodiment, the composition of the present invention is in the form of a topical composition, which includes a topical carrier. Preferably, the topical carrier is selected from a broad range of traditional personal care carriers depending on the type of composition to be formed. By suitable selections of compatible carriers, it is contemplated that such a composition is prepared in the form of daily skin or hair products including conditioning treatments, cleansing products, such as hair and/or scalp shampoos, body washes, hand cleansers, water-less hand sanitizer/cleansers, facial cleansers and the like.

In a preferred embodiment, the carrier is water. Preferably the compositions of the present invention comprise from 40% to 95% water by weight of the composition; preferably from 50% to 85%, more preferably still from 60% to 80%.

D. Detersive Surfactant

The composition of the present invention includes a detersive surfactant. The detersive surfactant component is included to provide cleaning performance to the composition. The detersive surfactant component in turn comprises anionic detersive surfactant, zwitterionic or amphoteric detersive surfactant, or a combination thereof. Such surfactants should be physically and chemically compatible with the essential components described herein, or should not otherwise unduly impair product stability, aesthetics or performance.

Suitable anionic detersive surfactant components for use in the composition herein include those which are known for use in hair care or other personal care cleansing compositions. The concentration of the anionic surfactant component in the composition should be sufficient to provide the desired cleaning and lather performance, and generally range from about 5% to about 50%, preferably from about 8% to about 30%, more preferably from about 10% to about

25%, even more preferably from about 12% to about 22%.

Preferred anionic surfactants suitable for use in the compositions are the alkyl and alkyl ether sulfates. These materials have the respective formulae $ROSO_3M$ and $RO(C_2H_4O)_XSO_3M$, wherein R is alkyl or alkenyl of from about 8 to about 18 carbon atoms, x is an integer having a value of from 1 to 10, and M is a cation such as ammonium, alkanolamines, such as triethanolamine, monovalent metals, such as sodium and potassium, and polyvalent metal cations, such as magnesium, and calcium.

Preferably, R has from about 8 to about 18 carbon atoms, more preferably from about 10 to about 16 carbon atoms, even more preferably from about 12 to about 14 carbon atoms, in both the alkyl and alkyl ether sulfates. The alkyl ether sulfates are typically made as condensation products of ethylene oxide and monohydric alcohols having from about 8 to about 24 carbon atoms. The alcohols can be synthetic or they can be derived from fats, e.g., coconut oil, palm kernel oil, tallow. Lauryl alcohol and straight chain alcohols derived from coconut oil or palm kernel oil are preferred. Such alcohols are reacted with between about 0 and about 10, preferably from about 2 to about 5, more preferably about 3, molar proportions of ethylene oxide, and the resulting mixture of molecular species having, for example, an average of 3 moles of ethylene oxide per mole of alcohol, is sulfated and neutralized.

Other suitable anionic detersive surfactants are the water-soluble salts of organic, sulfuric acid reaction products conforming to the formula [ $R^1$-$SO_3$-M ] where $R^1$ is a straight or branched chain, saturated, aliphatic hydrocarbon radical having from about 8 to about 24, preferably about 10 to about 18, carbon atoms; and M is a cation described hereinbefore.

Still other suitable anionic detersive surfactants are the reaction products of fatty acids esterified with isethionic acid and neutralized with sodium hydroxide where, for example, the fatty acids are derived from coconut oil or palm kernel oil; sodium or potassium salts of fatty acid amides of methyl tauride in which the fatty acids, for example, are derived from coconut oil or palm kernel oil. Other similar anionic surfactants are described in U.S. Pat. Nos. 2,486,921; 2,486,922; and 2,396,278.

Other anionic detersive surfactants suitable for use in the compositions are the succinnates, examples of which include disodium N-octadecylsulfosuccinnate; disodium lauryl sulfosuccinate; diammonium lauryl; tetrasodium N-(1,2-dicarboxyethyl)-N-octadecylsulfosuccinnate; diamyl ester of sodium sulfosuccinic acid; dihexyl ester of sodium sulfosuccinic acid; and dioctyl esters of sodium sulfosuccinic acid.

Other suitable anionic detersive surfactants include olefin sulfonates having about 10 to about 24 carbon atoms. In addition to the true alkene sulfonates and a proportion of hydroxy-alkanesulfonates, the olefin sulfonates can contain minor amounts of other materials, such as alkene disulfonates depending upon the reaction conditions, proportion of reactants, the nature of the starting olefins and impurities in the olefin stock and side reactions during the sulfonation process. A non limiting example of such an alpha-olefin sulfonate mixture is described in U.S. Patent 3,332,880.

Another class of anionic detersive surfactants suitable for use in the compositions are the beta-alkyloxy alkane sulfonates. These surfactants conform to the formula:

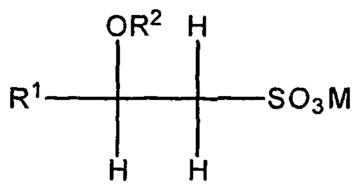

where $R^1$ is a straight chain alkyl group having from about 6 to about 20 carbon atoms, $R^2$ is a lower alkyl group having from about 1 to about 3 carbon atoms, preferably 1 carbon atom, and M is a water-soluble cation as described hereinbefore.

Preferred anionic detersive surfactants for use in the compositions include ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, triethanolamine lauryl sulfate, triethanolamine lauryl sulfate, monoethanolamine cocoyl sulfate, monoethanolamine lauryl sulfate, sodium tridecyl benzene sulfonate, sodium dodecyl benzene sulfonate, sodium cocoyl isethionate and combinations thereof.

Suitable amphoteric or zwitterionic detersive surfactants for use in the composition herein include those, which are known for use in hair care or other personal care cleansing. Concentration of such amphoteric detersive surfactants preferably ranges from about 0.5% to about 20%, preferably from about 1% to about 10%. Non limiting examples of suitable zwitterionic or amphoteric surfactants are described in U.S. Pat. Nos. 5,104,646 (Bolich Jr. et al.), 5,106,609

(Bolich Jr. et al.).

Amphoteric detersive surfactants suitable for use in the composition are well known in the art, and include those surfactants broadly described as derivatives of aliphatic secondary and tertiary amines in which the aliphatic radical can be straight or branched chain and wherein one of the aliphatic substituents contains from about 8 to about 18 carbon atoms and one contains an anionic group such as carboxy, sulfonate, sulfate, phosphate, or phosphonate. Preferred amphoteric detersive surfactants for use in the present invention include cocoamphoacetate, cocoamphodiacetate, lauroamphoacetate, lauroamphodiacetate, and mixtures thereof.

Zwitterionic detersive surfactants suitable for use in the composition are well known in the art, and include those surfactants broadly described as derivatives of aliphatic quatemaryammonium, phosphonium, and sulfonium compounds, in which the aliphatic radicals can be straight or branched chain, and wherein one of the aliphatic substituents contains from about 8 to about 18 carbon atoms and one contains an anionic group such as carboxy, sulfonate, sulfate, phosphate or phosphonate. Zwitterionics such as betaines are preferred.

The compositions of the present invention may further comprise additional surfactants for use in combination with the anionic detersive surfactant component described hereinbefore. Suitable optional surfactants include nonionic and cationic surfactants. Any such surfactant known in the art for use in hair or personal care products may be used, provided that the optional additional surfactant is also chemically and physically compatible with the essential components of the composition, or does not otherwise unduly impair product performance, aesthetics or stability. The concentration of the optional additional surfactants in the composition may vary with the cleansing or lather performance desired, the optional surfactant selected, the desired product concentration, the presence of other components in the composition, and other factors well known in the art.

Non limiting examples of other anionic, zwitterionic, amphoteric or optional additional surfactants suitable for use in the compositions are described in McCutcheon's, Emulsifiers and Detergents, 1989 Annual, published by M. C. Publishing Co., and U.S. Pat. Nos. 3,929,678, 2,658,072; 2,438,091; 2,528,378.

E. Dispersed Particles

The composition of the present invention may include dispersed particles. In the compositions of the present invention, it is preferable to incorporate at least 0.025% by weight of the dispersed particles, more preferably at least 0.05%, still more preferably at least 0.1 %, even more preferably at least 0.25%, and yet more preferably at least 0.5% by weight of the dispersed particles. In the compositions of the present invention, it is preferable to incorporate no more than about 20% by weight of the dispersed particles, more preferably no more than about 10%, still more preferably no more than 5%, even more preferably no more than 3%, and yet more preferably no more than 2% by weight of the dispersed particles.

F. Aqueous Carrier

The compositions of the present invention are typically in the form of pourable liquids (under ambient conditions). The compositions will therefore typically comprise an aqueous carrier, which is present at a level of from about 20% to about 95%, preferably from about 60% to about 85%. The aqueous carrier may comprise water, or a miscible mixture of water and organic solvent, but preferably comprises water with minimal or no significant concentrations of organic solvent, except as otherwise incidentally incorporated into the composition as minor ingredients of other essential or optional components.

G. Additional Components

The compositions of the present invention may further comprise one or more optional components known for use in hair care or personal care products, provided that the optional components are physically and chemically compatible with the essential components described herein, or do not otherwise unduly impair product stability, aesthetics or performance. Individual concentrations of such optional components may range from about 0.001% to about 10%.

Non-limiting examples of optional components for use in the composition include cationic polymers, conditioning agents (hydrocarbon oils, fatty esters, silicones), anti dandruff agents, suspending agents, viscosity modifiers, dyes, nonvolatile solvents or diluents (water soluble and insoluble), pearlescent aids, foam boosters, additional surfactants or nonionic cosurfactants, pediculocides, pH adjusting agents, perfumes, preservatives, chelants, proteins, skin active agents, sunscreens, UV absorbers, and vitamins, minerals, herbal/fruit/food extracts, sphingolipids derivatives or synthetical derivative, and clay.

1. Cationic Polymers

The compositions of the present invention may contain a cationic polymer. Concentrations of the cationic polymer in the composition typically range from about 0.05% to about 3%, preferably from about 0.075% to about 2.0%, more preferably from about 0.1% to about 1.0%. Preferred cationic polymers will have cationic charge densities of at least about 0.9 meq/gm, preferably at least about 1.2 meq/gm, more preferably at least about 1.5 meq/gm, but also preferably less than about 7 meq/gm, more preferably less than about 5 meq/gm. Herein, "cationic charge density" of a polymer refers to the ratio of the number of positive charges on the polymer to the molecular weight of the polymer. The average molecular weight of such suitable cationic polymers will generally be between about 10,000 and 10 million, preferably between about 50,000 and about 5 million, more preferably between about 100,000 and about 3 million.

Suitable cationic polymers for use in the compositions of the present invention contain cationic nitrogen-containing moieties such as quaternary ammonium or cationic protonated amino moieties. The cationic protonated amines can be primary, secondary, or tertiary amines (preferably secondary or tertiary), depending upon the particular species and the selected pH of the composition. Any anionic counterions can be used in association with the cationic polymers so long as the polymers remain soluble in water, in the composition, or in a coacervate phase of the composition, and so long as the counterions are physically and chemically compatible with the essential components of the composition or do not otherwise unduly impair product performance, stability or aesthetics. Non limiting examples of such counterions include halides (e.g., chloride, fluoride, bromide, iodide), sulfate and methylsulfate.

Non limiting examples of such polymers are described in the CTFA Cosmetic Ingredient Dictionary, 3rd edition, edited by Estrin, Crosley, and Haynes, (The Cosmetic, Toiletry, and Fragrance Association, Inc., Washington, D.C. (1982)).

Non limiting examples of suitable cationic polymers include copolymers of vinyl monomers having cationic protonated amine or quaternary ammonium functionalities with water soluble spacer monomers such as acrylamide, methacrylamide, alkyl and dialkyl acrylamides, alkyl and dialkyl methacrylamides, alkyl acrylate, alkyl methacrylate, vinyl caprolactone or vinyl pyrrolidone.

Suitable cationic protonated amino and quaternary ammonium monomers, for inclusion in the cationic polymers of the composition herein, include vinyl compounds substituted with dialkylaminoalkyl acrylate, dialkylaminoalkyl methacrylate, monoalkylaminoalkyl acrylate, monoalkylaminoalkyl methacrylate, trialkyl methacryloxyalkyl ammonium salt, trialkyl acryloxyalkyl ammonium salt, diallyl quaternary ammonium salts, and vinyl quaternary ammonium monomers having cyclic cationic nitrogen-containing rings such as pyridinium, imidazolium, and quaternized pyrrolidone, e.g., alkyl vinyl imidazolium, alkyl vinyl pyridinium, alkyl vinyl pyrrolidone salts.

Other suitable cationic polymers for use in the compositions include copolymers of 1-vinyl-2-pyrrolidone and 1-vinyl-3-methylimidazolium salt (e.g., chloride salt) (referred to in the industry by the Cosmetic, Toiletry, and Fragrance Association, "CTFA", as Polyquaternium-16); copolymers of 1-vinyl-2-pyrrolidone and dimethylaminoethyl methacrylate (referred to in the industry by CTFA as Polyquaternium-11); cationic diallyl quaternary ammonium-containing polymers, including, for example, dimethyldiallylammonium chloride homopolymer, copolymers of acrylamide and dimethyldiallylammonium chloride (referred to in the industry by CTFA as Polyquaternium 6 and Polyquaternium 7, respectively); amphoteric copolymers of acrylic acid including copolymers of acrylic acid and dimethyldiallylammonium chloride (referred to in the industry by CTFA as Polyquaternium 22), terpolymers of acrylic acid with dimethyldiallylammonium chloride and acrylamide (referred to in the industry by CTFA as Polyquaternium 39), and terpolymers of acrylic acid with methacrylamidopropyl trimethylammonium chloride and methylacrylate (referred to in the industry by CTFA as Polyquaternium 47). Preferred cationic substituted monomers are the cationic substituted dialkylaminoalkyl acrylamides, dialkylaminoalkyl methacrylamides, and combinations thereof. These preferred monomers conform to the formula

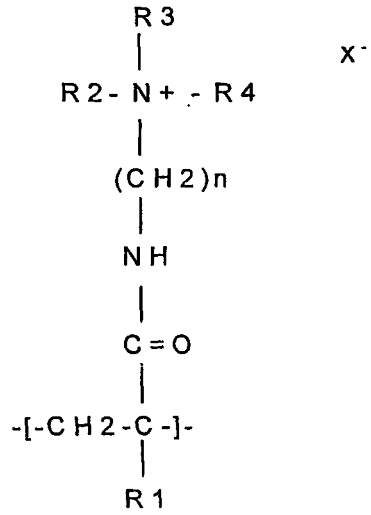

wherein $R^1$ is hydrogen, methyl or ethyl; each of $R^2$, $R^3$ and $R^4$ are independently hydrogen or a short chain alkyl having from about 1 to about 8 carbon atoms, preferably from about 1 to about 5 carbon atoms, more preferably from about 1 to about 2 carbon atoms; n is an integer having a value of from about 1 to about 8, preferably from about 1 to about 4; and X is a counterion. The nitrogen attached to $R^2$, $R^3$ and $R^4$ may be a protonated amine (primary, secondary or tertiary), but is preferably a quaternary ammonium wherein each of $R^2$, $R^3$ and $R^4$ are alkyl groups a non limiting example of which is polymethyacrylamidopropyl trimonium chloride, available under the trade name Polycare 133, from Rhone-Poulenc, Cranberry, N.J., U.S.A.

Other suitable cationic polymers for use in the composition include polysaccharide polymers, such as cationic cellulose derivatives and cationic starch derivatives. Suitable cationic polysaccharide polymers include those, which conform to the formula

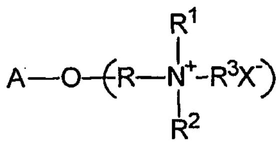

wherein A is an anhydroglucose residual group, such as a starch or cellulose anhydroglucose residual; R is an alkylene oxyalkylene, polyoxyalkylene, or hydroxyalkylene group, or combination thereof; R1, R2, and R3 independently are alkyl, aryl, alkylaryl, arylalkyl, alkoxyalkyl, or alkoxyaryl groups, each group containing up to about 18 carbon atoms, and the total number of carbon atoms for each cationic moiety (i.e., the sum of carbon atoms in R1, R2 and R3) preferably being about 20 or less; and X is an anionic counterion as described in hereinbefore.

Preferred cationic cellulose polymers are salts of hydroxyethyl cellulose reacted with trimethyl ammonium substituted epoxide, referred to in the industry (CTFA) as Polyquaternium 10 and available from Amerchol Corp. (Edison, N.J., USA) in their Polymer LR, JR, and KG series of polymers. Other suitable types of cationic cellulose includes the polymeric quaternary ammonium salts of hydroxyethyl cellulose reacted with lauryl dimethyl ammonium-substituted epoxide referred to in the industry (CTFA) as Polyquaternium 24. These materials are available from Amerchol Corp. under the tradename Polymer LM-200.

Other suitable cationic polymers include cationic guar gum derivatives, such as guar hydroxypropyltrimonium chloride, specific examples of which include the Jaguar series commercially available from Rhone-Poulenc Incorporated and the N-Hance series commercially available from Aqualon Division of Hercules, Inc. Other suitable cationic polymers include quaternary nitrogen-containing cellulose ethers, some examples of which are described in U.S. Pat. No. 3,962,418. Other suitable cationic polymers include copolymers of etherified cellulose, guar and starch, some examples of which are described in U.S. Pat. No. 3,958,581. When used, the cationic polymers herein are either soluble in the composition or are soluble in a complex coacervate phase in the composition formed by the cationic polymer and the anionic, amphoteric and/or zwitterionic detersive surfactant component described hereinbefore. Complex coacervates of the cationic polymer can also be formed with other charged materials in the composition.

Techniques for analysis of formation of complex coacervates are known in the art.

For example, microscopic analyses of the compositions, at any chosen stage of dilution, can be utilized to identify whether a coacervate phase has formed. Such coacervate phase will be identifiable as an additional emulsified phase in the composition. The use of dyes can aid in distinguishing the coacervate phase from other insoluble phases dispersed in the composition.

2. Nonionic polymers

Polyalkylene glycols having a molecular weight of more than about 1000 are useful herein. Useful are those having the following general formula:

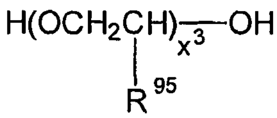

wherein $R^{95}$ is selected from the group consisting of H, methyl, and mixtures thereof. Polyethylene glycol polymers useful herein are PEG-2M (also known as Polyox WSR® N-10, which is available from Union Carbide and as PEG-2,000); PEG-5M (also known as Polyox WSR® N-35 and Polyox WSR® N-80, available from Union Carbide and as PEG-5,000 and Polyethylene Glycol 300,000); PEG-7M (also known as Polyox WSR® N-750 available from Union Carbide); PEG-9M (also known as Polyox WSR® N-3333 available from Union Carbide); and PEG-14 M (also known as Polyox WSR® N-3000 available from Union Carbide).

3. Conditioning agents

Conditioning agents include any material, which is used to give a particular conditioning benefit to hair and/or skin. In hair treatment compositions, suitable conditioning agents are those which deliver one or more benefits relating to shine, softness, combability, antistatic properties, wet-handling, damage, manageability, body, and greasiness. The conditioning agents useful in the compositions of the present invention typically comprise a water insoluble, water dispersible, non-volatile, liquid that forms emulsified, liquid particles. Suitable conditioning agents for use in the composition are those conditioning agents characterized generally as silicones (e.g., silicone oils, cationic silicones, silicone gums, high refractive silicones, and silicone resins), organic conditioning oils (e.g., hydrocarbon oils, polyolefins, and fatty esters) or combinations thereof, or those conditioning agents which otherwise form liquid, dispersed particles in the aqueous surfactant matrix herein. Such conditioning agents should be physically and chemically compatible with the essential components of the composition, and should not otherwise unduly impair product stability, aesthetics or performance.

The concentration of the conditioning agent in the composition should be sufficient to provide the desired conditioning benefits, and as will be apparent to one of ordinary skill in the art. Such concentration can vary with the conditioning agent, the conditioning performance desired, the average size of the conditioning agent particles, the type and concentration of other components, and other like factors.

1. Silicones

The conditioning agent of the compositions of the present invention is preferably an insoluble silicone conditioning agent. The silicone conditioning agent particles may comprise volatile silicone, non-volatile silicone, or combinations thereof. Preferred are non-volatile silicone conditioning agents. If volatile silicones are present, it will typically be incidental to their use as a solvent or carrier for commercially available forms of non-volatile silicone materials ingredients, such as silicone gums and resins. The silicone conditioning agent particles may comprise a silicone fluid conditioning agent and may also comprise other ingredients, such as a silicone resin to improve silicone fluid deposition efficiency or enhance glossiness of the hair.

The concentration of the silicone conditioning agent typically ranges from about 0.01% to about 10%, preferably from about 0.1% to about 8%, more preferably from about 0.1% to about 5%, more preferably from about 0.2% to about 3%. Non-limiting examples of suitable silicone conditioning agents, and optional suspending agents for the silicone, are described in U.S. Reissue Pat. No. 34,584, U.S. Pat. No. 5,104,646, and U.S. Pat. No. 5,106,609. The silicone conditioning agents for use in the compositions of the present invention preferably have a viscosity, as measured at 25°C, from about 20 to about 2,000,000 centistokes ("csk"), more preferably from about 1,000 to about 1,800,000 csk, even more preferably from about 50,000 to about 1,500,000 csk, more preferably from about 100,000 to about 1,500,000 csk.

The dispersed silicone conditioning agent particles typically have a volume average particle diameter ranging

from about 0.01μm to about 50μm, as measured using the Horiba LA-910 Particle Size Analyzer. The Horiba LA-910 instrument uses the principles of low-angle Fraunhofer Diffraction and Light Scattering to measure the particle size and distribution in a dilute solution of particles. For small particle application to hair, the volume average particle diameters typically range from about 0.01μm to about 4μm, preferably from about 0.01μm to about 2μm, more preferably from about 0.01μm to about 0.5μm. For larger particle application to hair, the volume average particle diameters typically range from about 4μm to about 50μm, preferably from about 6μm to about 40μm, and more preferably from about 10μm to about 35μm.

Background material on silicones including sections discussing silicone fluids, gums, and resins, as well as manufacture of silicones, are found in Encyclopedia of Polymer Science and Engineering, vol. 15, 2d ed., pp 204-308, John Wiley & Sons, Inc. (1989).

a. Silicone oils

Silicone fluids include silicone oils, which are flowable silicone materials having a viscosity, as measured at 25°C, less than 1,000,000 csk, preferably from about 5 csk to about 1,000,000 csk, more preferably from about 100 csk to about 600,000 csk. Suitable silicone oils for use in the compositions of the present invention include polyalkyl siloxanes, polyaryl siloxanes, polyalkylaryl siloxanes, polyether siloxane copolymers, and mixtures thereof. Other insoluble; non-volatile silicone fluids having hair conditioning properties may also be used.

Silicone oils include polyalkyl or polyaryl siloxanes which conform to the following Formula (III):

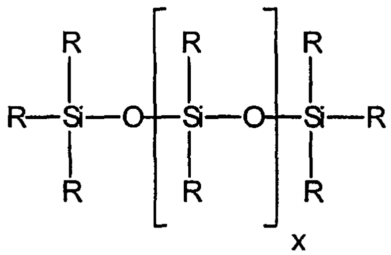

wherein R is aliphatic, preferably alkyl or alkenyl, or aryl, R can be substituted or unsubstituted, and x is an integer from 1 to about 8,000. Suitable R groups for use in the compositions of the present invention include, but are not limited to: alkoxy, aryloxy, alkaryl, arylalkyl, arylalkenyl, alkamino, and ether-substituted, hydroxyl-substituted, and halogen-substituted aliphatic and aryl groups. Suitable R groups also include cationic amines and quaternary ammonium groups.

Preferred alkyl and alkenyl substituents are $C_1$ to $C_5$ alkyls and alkenyls, more preferably from $C_1$ to $C_4$, more preferably from $C_1$ to $C_2$. The aliphatic portions of other alkyl-, alkenyl-, or alkynyl-containing groups (such as alkoxy, alkaryl, and alkamino) can be straight or branched chains, and are preferably from $C_1$ to $C_5$, more preferably from $C_1$ to $C_4$, even more preferably from $C_1$ to $C_3$, more preferably from $C_1$ to $C_2$. As discussed above, the R substituents can also contain amino functionalities (e.g. alkamino groups), which can be primary, secondary or tertiary amines or quaternary ammonium. These include mono-, di- and tri- alkylamino and alkoxyamino groups, wherein the aliphatic portion chain length is preferably as described herein.

b. Amino and Cationic silicones

Cationic silicone fluids suitable for use in the compositions of the present invention include, but are not limited to, those which conform to the general formula (V):

$$R_1)_aG_{3-a}\text{-Si-}(\text{-OSiG}_2)_n\text{-}(\text{-OSiG}_b(R_1)_{2-b)m}\text{-O-SiG}_{3-a}(R_1)_a$$

wherein G is hydrogen, phenyl, hydroxy, or $C_1$-$C_8$ alkyl, preferably methyl; a is 0 or an integer having a value from 1 to 3, preferably 0; b is 0 or 1, preferably 1; n is a number from 0 to 1,999, preferably from 49 to 499; m is an integer from 1 to 2,000, preferably from 1 to 10; the sum of n and m is a number from 1 to 2,000, preferably from 50 to 500; $R_1$ is a monovalent radical conforming to the general formula $CqH_{2q}L$, wherein q is an integer having a value from 2 to 8 and L is selected from the following groups:

$-N(R_2)CH_2-CH_2-N(R_2)_2$

$-N(R_2)_2$

$-N(R_2)_3A^-$

$-N(R_2)CH_2-CH_2-NR_2H_2A^-$

wherein $R_2$ is hydrogen, phenyl, benzyl, or a saturated hydrocarbon radical, preferably an alkyl radical from about $C_1$ to about $C_{20}$, and $A^-$ is a halide ion.

An especially preferred cationic silicone corresponding to formula (V) is the polymer known as "trimethylsilylamodimethicone", which is shown below in formula (VI):

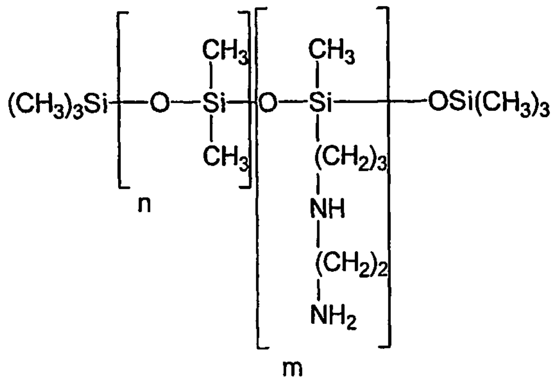

Other silicone cationic polymers which may be used in the compositions of the present invention are represented by the general formula (VII):

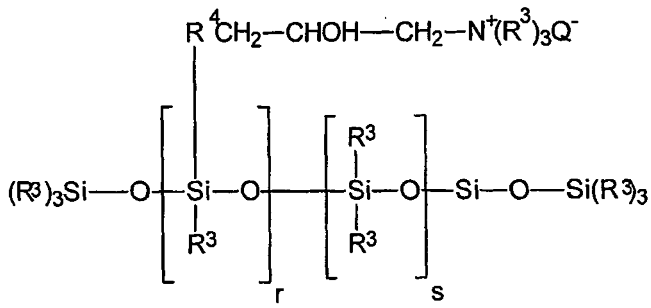

wherein $R^3$ is a monovalent hydrocarbon radical from $C_1$ to $C_{18}$, preferably an alkyl or alkenyl radical, such as methyl; $R_4$ is a hydrocarbon radical, preferably a $C_1$ to $C_{18}$ alkylene radical or a $C_{10}$ to $C_{18}$ alkyleneoxy radical, more preferably a $C_1$ to $C_8$ alkyleneoxy radical; $Q^-$ is a halide ion, preferably chloride; r is an average statistical value from 2 to 20, preferably from 2 to 8; s is an average statistical value from 20 to 200, preferably from 20 to 50. A preferred polymer of this class is known as UCARE SILICONE ALE 56™, available from Union Carbide.

c. Silicone gums

Other silicone fluids suitable for use in the compositions of the present invention are the insoluble silicone gums. These gums are polyorganosiloxane materials having a viscosity, as measured at 25°C, of greater than or equal to 1,000,000 csk. Silicone gums are described in U.S. Pat. No. 4,152,416; Noll and Walter, Chemistry and Technology of Silicones, New York: Academic Press (1968); and in General Electric Silicone Rubber Product Data Sheets SE 30, SE 33, SE 54 and SE 76. Specific non-limiting examples of silicone gums for use in the compositions of the present invention include polydimethylsiloxane, (polydimethylsiloxane) (methylvinylsiloxane) copolymer, poly(dimethylsiloxane) (diphenyl siloxane)(methylvinylsiloxane) copolymer and mixtures thereof.

d. High refractive index silicones

Other non-volatile, insoluble silicone fluid conditioning agents that are suitable for use in the compositions of

the present invention are those known as "high refractive index silicones," having a refractive index of at least about 1.46, preferably at least about 1.48, more preferably at least about 1.52, more preferably at least about 1.55. The refractive index of the polysiloxane fluid will generally be less than about 1.70, typically less than about 1.60. In this context, polysiloxane "fluid" includes oils as well as gums.

The high refractive index polysiloxane fluid includes those represented by general Formula (III) above, as well as cyclic polysiloxanes such as those represented by Formula (VIII) below:

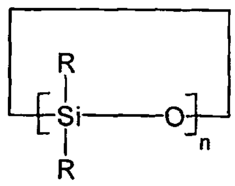

wherein R is as defined above, and n is a number from about 3 to about 7, preferably from about 3 to about 5.

The high refractive index polysiloxane fluids contain an amount of aryl-containing R substituents sufficient to increase the refractive index to the desired level, which is described herein. Additionally, R and n must be selected so that the material is non-volatile.

Aryl-containing substituents include those which contain alicyclic and heterocyclic five and six member aryl rings and those which contain fused five or six member rings. The aryl rings themselves can be substituted or unsubstituted.

Generally, the high refractive index polysiloxane fluids will have a degree of aryl-containing substituents of at least about 15%, preferably at least about 20%, more preferably at least about 25%, even more preferably at least about 35%, most preferably at least about 50%. Typically, the degree of aryl substitution will be less than about 90%, more generally less than about 85%, preferably from about 55% to about 80%.

Preferred high refractive index polysiloxane fluids have a combination of phenyl or phenyl derivative substituents (more preferably phenyl), with alkyl substituents, preferably $C_1$-$C_4$ alkyl (more preferably methyl), hydroxy, or $C_1$-$C_4$ alkylamino (especially -$R^1NHR^2NH2$ wherein each $R^1$ and $R^2$ independently is a $C_1$-$C_3$ alkyl, alkenyl, and/or alkoxy).

When high refractive index silicones are used in the compositions of the present invention, they are preferably used in solution with a spreading agent, such as a silicone resin or a surfactant, to reduce the surface tension by a sufficient amount to enhance spreading and thereby enhance the glossiness (subsequent to drying) of hair treated with the compositions.

Silicone fluids suitable for use in the compositions of the present invention are disclosed in U.S. Pat. No. 2,826,551, U.S. Pat. No. 3,964,500, U.S. Pat. No. 4,364,837, British Pat. No. 849,433, and Silicon Compounds, Petrarch Systems, Inc. (1984).

e. Silicone resins

Silicone resins may be included in the silicone conditioning agent of the compositions of the present invention. These resins are highly cross-linked polymeric siloxane systems. The cross-linking is introduced through the incorporation of trifunctional and tetrafunctional silanes with monofunctional or difunctional, or both, silanes during manufacture of the silicone resin.

Silicone materials and silicone resins in particular, can conveniently be identified according to a shorthand nomenclature system known to those of ordinary skill in the art as "MDTQ" nomenclature. Under this system, the silicone is described according to presence of various siloxane monomer units which make up the silicone. Briefly, the symbol M denotes the monofunctional unit $(CH_3)_3SiO_{0.5}$; D denotes the difunctional unit $(CH_3)_2SiO$; T denotes the trifunctional unit $(CH_3)SiO_{1.5}$; and Q denotes the quadra- or tetra-functional unit $SiO_2$. Primes of the unit symbols (e.g. M', D', T', and Q') denote substituents other than methyl, and must be specifically defined for each occurrence.

Preferred silicone resins for use in the compositions of the present invention include, but are not limited to MQ, MT, MTQ, MDT and MDTQ resins. Methyl is a preferred silicone substituent. Especially preferred silicone resins are MQ resins, wherein the M:Q ratio is from about 0.5:1.0 to about 1.5:1.0 and the average molecular weight of the silicone resin is from about 1000 to about 10,000.

The weight ratio of the non-volatile silicone fluid, having refractive index below 1.46, to the silicone resin component, when used, is preferably from about 4:1 to about 400:1, more preferably from about 9:1 to about 200:1, more preferably from about 19:1 to about 100:1, particularly when the silicone fluid component is a polydimethylsiloxane fluid or a mixture of polydimethylsiloxane fluid and polydimethylsiloxane gum as described herein. Insofar as the silicone resin forms a part of the same phase in the compositions hereof as the silicone fluid, i.e. the conditioning active, the

sum of the fluid and resin should be included in determining the level of silicone conditioning agent in the composition.

2. Organic conditioning oils

The conditioning component of the compositions of the present invention may also comprise from about 0.05% to about 3%, preferably from about 0.08% to about 1.5%, more preferably from about 0.1% to about 1%, of at least one organic conditioning oil as the conditioning agent, either alone or in combination with other conditioning agents, such as the silicones (described herein).

a. Hydrocarbon oils

Suitable organic conditioning oils for use as conditioning agents in the compositions of the present invention include, but are not limited to, hydrocarbon oils having at least about 10 carbon atoms, such as cyclic hydrocarbons, straight chain aliphatic hydrocarbons (saturated or unsaturated), and branched chain aliphatic hydrocarbons (saturated or unsaturated), including polymers and mixtures thereof. Straight chain hydrocarbon oils preferably are from about $C_{12}$ to about $C_{19}$. Branched chain hydrocarbon oils, including hydrocarbon polymers, typically will contain more than 19 carbon atoms.

Specific non-limiting examples of these hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, polybutene, polydecene, and mixtures thereof. Branched-chain isomers of these compounds, as well as of higher chain length hydrocarbons, can also be used, examples of which include highly branched, saturated or unsaturated, alkanes such as the permethyl-substituted isomers, e.g., the permethyl-substituted isomers of hexadecane and eicosane, such as 2, 2, 4, 4, 6, 6, 8, 8-dimethyl-10-methylundecane and 2, 2, 4, 4, 6, 6-dimethyl-8-methylnonane, available from Permethyl Corporation. Hydrocarbon polymers such as polybutene and polydecene. A preferred hydrocarbon polymer is polybutene, such as the copolymer of isobutylene and butene. A commercially available material of this type is L-14 polybutene from Amoco Chemical Corporation. The concentration of such hydrocarbon oils in the composition preferably range from about 0.05% to about 20%, more preferably from about 0.08% to about 1.5%, and even more preferably from about 0.1 % to about 1%.

b. Polyolefins

Organic conditioning oils for use in the compositions of the present invention can also include liquid polyolefins, more preferably liquid poly-α-olefins, more preferably hydrogenated liquid poly-α-olefins. Polyolefins for use herein are prepared by polymerization of $C_4$ to about $C_{14}$ olefenic monomers, preferably from about $C_6$ to about $C_{12}$.

Non-limiting examples of olefenic monomers for use in preparing the polyolefin liquids herein include ethylene, propylene, 1-butene, 1-pentene, 1-hexene, 1-octene, 1-decene, 1-dodecene, 1-tetradecene, branched chain isomers such as 4-methyl-1-pentene, and mixtures thereof. Also suitable for preparing the polyolefin liquids are olefin-containing refinery feedstocks or effluents. Preferred hydrogenated α-olefin monomers include, but are not limited to: 1-hexene to 1-hexadecenes, 1-octene to 1-tetradecene, and mixtures thereof.

c. Fatty Esters

Other suitable organic conditioning oils for use as the conditioning agent in the compositions of the present invention include, but are not limited to, fatty esters having at least 10 carbon atoms. These fatty esters include esters with hydrocarbyl chains derived from fatty acids or alcohols (e.g. mono-esters, polyhydric alcohol esters, and di- and tricarboxylic acid esters). The hydrocarbyl radicals of the fatty esters hereof may include or have covalently bonded thereto other compatible functionalities, such as amides and alkoxy moieties (e.g., ethoxy or ether linkages, etc.).

Specific examples of preferred fatty esters include, but are not limited to: isopropyl isostearate, hexyl laurate, isohexyl laurate, isohexyl palmitate, isopropyl palmitate, decyl oleate, isodecyl oleate, hexadecyl stearate, decyl stearate, isopropyl isostearate, dihexyldecyl adipate, lauryl lactate, myristyl lactate, cetyl lactate, oleyl stearate, oleyl oleate, oleyl myristate, lauryl acetate, cetyl propionate, and oleyl adipate.

Other fatty esters suitable for use in the compositions of the present invention are mono-carboxylic acid esters of the general formula R'COOR, wherein R' and R are alkyl or alkenyl radicals, and the sum of carbon atoms in R' and R is at least 10, preferably at least 22.

Still other fatty esters suitable for use in the compositions of the present invention are di- and tri-alkyl and alkenyl esters of carboxylic acids, such as esters of $C_4$ to $C_8$ dicarboxylic acids (e.g. $C_1$ to $C_{22}$ esters, preferably $C_1$ to $C_6$, of succinic acid, glutaric acid, and adipic acid). Specific non-limiting examples of di- and tri- alkyl and alkenyl esters of carboxylic acids include isocetyl stearyol stearate, diisopropyl adipate, and tristearyl citrate.

Other fatty esters suitable for use in the compositions of the present invention are those known as polyhydric alcohol esters. Such polyhydric alcohol esters include alkylene glycol esters, such as ethylene glycol mono and di-fatty acid esters, diethylene glycol mono- and di-fatty acid esters, polyethylene glycol mono- and di-fatty acid esters, propylene glycol mono- and di-fatty acid esters, polypropylene glycol monooleate, polypropylene glycol 2000 monostearate, ethoxylated propylene glycol monostearate, glyceryl mono- and di-fatty acid esters, polyglycerol poly-fatty acid esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters.

Still other fatty esters suitable for use in the compositions of the present invention are glycerides, including, but not limited to, mono-, di-, and tri-glycerides, preferably di- and tri-glycerides, more preferably triglycerides. For use in the compositions described herein, the glycerides are preferably the mono-, di-, and tri-esters of glycerol and long chain carboxylic acids, such as $C_{10}$ to $C_{22}$ carboxylic acids. A variety of these types of materials can be obtained from vegetable and animal fats and oils, such as castor oil, safflower oil, cottonseed oil, corn oil, olive oil, cod liver oil, almond oil, avocado oil, palm oil, sesame oil, lanolin and soybean oil. Synthetic oils include, but are not limited to, triolein and tristearin glyceryl dilaurate.

Other fatty esters suitable for use in the compositions of the present invention are water insoluble synthetic fatty esters. Some preferred synthetic esters conform to the general Formula (IX):

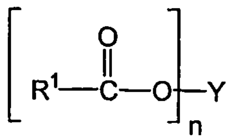

wherein $R^1$ is a $C_7$ to $C_9$ alkyl, alkenyl, hydroxyalkyl or hydroxyalkenyl group, preferably a saturated alkyl group, more preferably a saturated, linear, alkyl group; n is a positive integer having a value from 2 to 4, preferably 3; and Y is an alkyl, alkenyl, hydroxy or carboxy substituted alkyl or alkenyl, having from about 2 to about 20 carbon atoms, preferably from about 3 to about 14 carbon atoms. Other preferred synthetic esters conform to the general Formula (X):

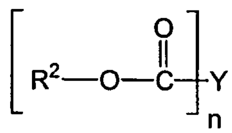

wherein $R^2$ is a $C_8$ to $C_{10}$ alkyl, alkenyl, hydroxyalkyl or hydroxyalkenyl group; preferably a saturated alkyl group, more preferably a saturated, linear, alkyl group; n and Y are as defined above in Formula (X).

Specific non-limiting examples of suitable synthetic fatty esters for use in the compositions of the present invention include: P-43 ($C_8$-$C_{10}$ triester of trimethylolpropane), MCP-684 (tetraester of 3,3 diethanol-1,5 pentadiol), MCP 121 ($C_8$-$C_{10}$ diester of adipic acid), all of which are available from Mobil Chemical Company.

3. Other conditioning agents

Also suitable for use in the compositions herein are the conditioning agents described by the Procter & Gamble Company in U.S. Pat. Nos. 5,674,478, and 5,750,122. Also suitable for use herein are those conditioning agents described in U.S. Pat. Nos. 4,529,586 (Clairol), 4,507,280 (Clairol), 4,663,158 (Clairol), 4,197,865 (L'Oreal), 4,217, 914 (L'Oreal), 4,381,919 (L'Oreal), and 4,422, 853 (L'Oreal).

4. Additional Components

The compositions of the present invention may further include a variety of additional useful components. Preferred additional components include those discussed below:

1. Other Anti-Microbial Actives

The compositions of the present invention may further include one or more antifungal or anti-microbial actives. In a preferred embodiment, the present invention may comprise pyrithione or a polyvalent metal salt of pyrithione. Any

form of polyvalent metal pyrithione salts may be used, including platelet and needle structures. Preferred salts for use herein include those formed from the polyvalent metals magnesium, barium, bismuth, strontium, copper, zinc, cadmium, zirconium and mixtures thereof, more preferably zinc. Even more preferred for use herein is the zinc salt of 1-hydroxy-2-pyridinethione (known as "zinc pyrithione" or "ZPT"); more preferably ZPT in platelet particle form, wherein the particles have an average size of up to about 20μm, preferably up to about 5μm, more preferably up to about 2.5μm. Preferred embodiments include 0.001% to about 10% of a pyrithione or polyvalent metal salt of a pyrithione; preferably from about 0.01% to about 5% of a pyrithione or polyvalent metal salt of a pyrithione; more preferably from about 0.1% to about 2%. In addition to the metal pyrithione salt actives suitable anti-microbial actives include coal tar, sulfur, whitfield's ointment, castellani's paint, aluminum chloride, gentian violet, octopirox (piroctone olamine), ciclopirox olamine, undecylenic acid and it's metal salts, potassium permanganate, selenium sulfide, sodium thiosulfate, propylene glycol, oil of bitter orange, urea preparations, griseofulvin, 8-Hydroxyquinoline ciloquinol, thiobendazole, thiocarbamates, haloprogin, polyenes, hydroxypyridone, morpholine, benzylamine, allylamines (such as terbinafine), tea tree oil, clove leaf oil, coriander, palmarosa, berberine, thyme red, cinnamon oil, cinnamic aldehyde, citronellic acid, hinokitol, ichthyol pale, Sensiva SC-50, Elestab HP-100, azelaic acid, lyticase, iodopropynyl butylcarbamate (IPBC), isothiazalinones such as octyl isothiazalinone and azoles, and combinations thereof. Preferred anti-microbials include itraconazole, ketoconazole, selenium sulphide and coal tar.

a. Azoles

Azole anti-microbials include imidazoles such as benzimidazole, benzothiazole, bifonazole, butaconazole nitrate, climbazole, clotrimazole, croconazole, eberconazole, econazole, elubiol, fenticonazole, fluconazole, flutimazole, isoconazole, ketoconazole, lanoconazole, metronidazole, miconazole, neticonazole, omoconazole, oxiconazole nitrate, sertaconazole, sulconazole nitrate, tioconazole, thiazole, and triazoles such as terconazole and itraconazole, and combinations thereof. When present in the composition, the azole anti-microbial active is included in an amount from about 0.01% to about 5%, preferably from about 0.1% to about 3%, and more preferably from about 0.3% to about 2%, by weight of the composition. Especially preferred herein is ketoconazole.

b. Selenium Sulfide

Selenium sulfide is a particulate anti-dandruff agent suitable for use in the antimicrobial compositions of the present invention, effective concentrations of which range from about 0.1 % to about 4%, by weight of the composition, preferably from about 0.3% to about 2.5%, more preferably from about 0.5% to about 1.5%. Selenium sulfide is generally regarded as a compound having one mole of selenium and two moles of sulfur, although it may also be a cyclic structure that conforms to the general formula $Se_xS_y$,
wherein x + y = 8. Average particle diameters for the selenium sulfide are typically less than 15μm, as measured by forward laser light scattering device (e.g. Malvern 3600 instrument), preferably less than 10 μm. Selenium sulfide compounds are described, for example, in U.S. Pat. No. 2,694,668; U.S. Pat. No. 3,152,046; U.S. Pat. No. 4,089,945; and U.S. Pat. No. 4,885,107.

c. Sulfur

Sulfur may also be used as a particulate anti-microbial/anti-dandruff agent in the anti-microbial compositions of the present invention. Effective concentrations of the particulate sulfur are typically from about 1% to about 4%, by weight of the composition, preferably from about 2% to about 4%.

d. Keratolytic Agents

The present invention may further comprise one or more keratolytic agents such as Salicylic Acid.

Additional anti-microbial actives of the present invention may include extracts of melaleuca (tea tree) and charcoal. The present invention may also comprise combinations of anti-microbial actives. Such combinations may include octopirox and zinc pyrithione combinations, pine tar and sulfur combinations, salicylic acid and zinc pyrithione combinations, octopirox and climbasole combinations, and salicylic acid and octopirox combinations, and mixtures thereof.

2. Hair loss prevention and Hair Growth Agents

The present invention may further comprise materials useful for hair loss prevention and hair growth stimulants or agents. Examples of such agents are AntiAndrogens such as Propecia, Dutasteride, RU5884; Anti-Inflammatories

such as Glucocortisoids, Macrolides, Macrolides; Anti-Microbials such as Zinc pyrithione, Ketoconazole, Acne Treatments; Immunosuppressives such as FK-506, Cyclosporin; Vasodilators such as minoxidil, Aminexil® and combinations thereof.

3. Sensates

The present invention may further comprise topical sensate materials such as terpenes, vanilloids, alkyl amides, natural extracts and combinations thereof. Terpenes can include menthol and derivatives such as menthyl lactate, ethyl menthane carboxamide, and menthoyxypropanediol. Other terpenes can include camphor, eucalyptol, carvone, thymol and combinations thereof. Vanilloids can include capsaicin, zingerone, eugenol, and vanillyl butyl ether. Alkyl amides can include spilanthol, hydroxy alpha-sanschool, pellitorine and combinations thereof. Natural extracts can include peppermint oil, eucalyptol, rosemary oil, ginger oil, clove oil, capsicum, jambu extract, cinnamon oil, laricyl and combinations thereof. Additional topical sensate materials can include methyl salicylate, anethole, benzocaine, lidocane, phenol, benzyl nicotinate, nicotinic acid, cinnamic aldehyde, cinnamyl alcohol, piperine, and combinations thereof.

4. Humectant

The compositions of the present invention may contain a humectant. The humectants herein are selected from the group consisting of polyhydric alcohols, water soluble alkoxylated nonionic polymers, and mixtures thereof. The humectants, when used herein, are preferably used at levels of from about 0.1% to about 20%, more preferably from about 0.5% to about 5%.

Polyhydric alcohols useful herein include glycerin, sorbitol, propylene glycol, butylene glycol, hexylene glycol, ethoxylated glucose, 1, 2-hexane diol, hexanetriol, dipropylene glycol, erythritol, trehalose, diglycerin, xylitol, maltitol, maltose, glucose, fructose, sodium chondroitin sulfate, sodium hyaluronate, sodium adenosine phosphate, sodium lactate, pyrrolidone carbonate, glucosamine, cyclodextrin, and mixtures thereof.

Water soluble alkoxylated nonionic polymers useful herein include polyethylene glycols and polypropylene glycols having a molecular weight of up to about 1000 such as those with CTFA names PEG-200, PEG-400, PEG-600, PEG-1000, and mixtures thereof.

5. Suspending Agent

The compositions of the present invention may further comprise a suspending agent at concentrations effective for suspending water-insoluble material in dispersed form in the compositions or for modifying the viscosity of the composition. Such concentrations range from about 0.1% to about 10%, preferably from about 0.3% to about 5.0%.

Suspending agents useful herein include anionic polymers and nonionic polymers. Useful herein are vinyl polymers such as cross linked acrylic acid polymers with the CTFA name Carbomer, cellulose derivatives and modified cellulose polymers such as methyl cellulose, ethyl cellulose, hydroxyethyl cellulose, hydroxypropyl methyl cellulose, nitro cellulose, sodium cellulose sulfate, sodium carboxymethyl cellulose, crystalline cellulose, cellulose powder, polyvinylpyrrolidone, polyvinyl alcohol, guar gum, hydroxypropyl guar gum, xanthan gum, arabia gum, tragacanth, galactan, carob gum, guar gum, karaya gum, carragheenin, pectin, agar, quince seed (Cydonia oblonga Mill), starch (rice, corn, potato, wheat), algae colloids (algae extract), microbiological polymers such as dextran, succinoglucan, pulleran, starch-based polymers such as carboxymethyl starch, methylhydroxypropyl starch, alginic acid-based polymers such as sodium alginate, alginic acid propylene glycol esters, acrylate polymers such as sodium polyacrylate, polyethylacrylate, polyacrylamide, polyethyleneimine, and inorganic water soluble material such as bentonite, aluminum magnesium silicate, laponite, hectonite, and anhydrous silicic acid.

Commercially available viscosity modifiers highly useful herein include Carbomers with tradenames Carbopol 934, Carbopol 940, Carbopol 950, Carbopol 980, and Carbopol 981, all available from B. F. Goodrich Company, acrylates/steareth-20 methacrylate copolymer with tradename ACRYSOL 22 available from Rohm and Hass, nonoxynyl hydroxyethylcellulose with tradename AMERCELL POLYMER HM-1500 available from Amerchol, methylcellulose with tradename BENECEL, hydroxyethyl cellulose with tradename NATROSOL, hydroxypropyl cellulose with tradename KLUCEL, cetyl hydroxyethyl cellulose with tradename POLYSURF 67, all supplied by Hercules, ethylene oxide and/or propylene oxide based polymers with tradenames CARBOWAX PEGs, POLYOX WASRs, and UCON FLUIDS, all supplied by Amerchol.

Other optional suspending agents include crystalline suspending agents which can be categorized as acyl derivatives, long chain amine oxides, and mixtures thereof. These suspending agents are described in U.S. Pat. No. 4,741,855. These preferred suspending agents include ethylene glycol esters of fatty acids preferably having from about 16 to about 22 carbon atoms. More preferred are the ethylene glycol stearates, both mono and distearate, but particularly the distearate containing less than about 7% of the mono stearate. Other suitable suspending agents include alkanol

amides of fatty acids, preferably having from about 16 to about 22 carbon atoms, more preferably about 16 to 18 carbon atoms, preferred examples of which include stearic monoethanolamide, stearic diethanolamide, stearic monoisopropanolamide and stearic monoethanolamide stearate. Other long chain acyl derivatives include long chain esters of long chain fatty acids (e.g., stearyl stearate, cetyl palmitate, etc.); long chain esters of long chain alkanol amides (e.g., stearamide diethanolamide distearate, stearamide monoethanolamide stearate); and glyceryl esters (e.g., glyceryl distearate, trihydroxystearin, tribehenin) a commercial example of which is Thixin R available from Rheox, Inc. Long chain acyl derivatives, ethylene glycol esters of long chain carboxylic acids, long chain amine oxides, and alkanol amides of long chain carboxylic acids in addition to the preferred materials listed above may be used as suspending agents.

Other long chain acyl derivatives suitable for use as suspending agents include N,N-dihydrocarbyl amido benzoic acid and soluble salts thereof (e.g., Na, K), particularly N,N-di(hydrogenated) C.sub.16, C.sub.18 and tallow amido benzoic acid species of this family, which are commercially available from Stepan Company (Northfield, Ill., USA).

Examples of suitable long chain amine oxides for use as suspending agents include alkyl dimethyl amine oxides, e.g., stearyl dimethyl amine oxide.

Other suitable suspending agents include primary amines having a fatty alkyl moiety having at least about 16 carbon atoms, examples of which include palmitamine or stearamine, and secondary amines having two fatty alkyl moieties each having at least about 12 carbon atoms, examples of which include dipalmitoylamine or di(hydrogenated tallow) amine. Still other suitable suspending agents include di(hydrogenated tallow)phthalic acid amide, and crosslinked maleic anhydride-methyl vinyl ether copolymer.

6. Other Optional Components

The compositions of the present invention may contain also vitamins and amino acids such as: water soluble vitamins such as vitamin B1, B2, B6, B12, C, pantothenic acid, pantothenyl ethyl ether, panthenol, biotin, and their derivatives, water soluble amino acids such as asparagine, alanin, indole, glutamic acid and their salts, water insoluble vitamins such as vitamin A, D, E, and their derivatives, water insoluble amino acids such as tyrosine, tryptamine, and their salts.

The compositions of the present invention may also contain pigment materials such as inorganic, nitroso, monoazo, disazo, carotenoid, triphenyl methane, triaryl methane, xanthene, quinoline, oxazine, azine, anthraquinone, indigoid, thionindigoid, quinacridone, phthalocianine, botanical, natural colors, including: water soluble components such as those having C. I. Names. The compositions of the present invention may also contain antimicrobial agents which are useful as cosmetic biocides and antidandruff agents including: water soluble components such as piroctone olamine, water insoluble components such as 3,4,4'- trichlorocarbanilide (triclocarban), triclosan and zinc pyrithione.

The compositions of the present invention may also contain chelating agents.

H. pH

Preferably, the pH of the compositions of the present invention range from about 2 to about 11, preferably from about 4 to about 9, more preferably from about 6 to about 8.

Retained Activity of Diiodometyl-p-tolylsulfone in Dispersed Form

Biologically active materials must interact with their target on a molecular level to exert their effect. Materials with very low solubility (especially those wherein a major proportion exists as a particulate dispersion in w+ater) tend to have reduced activity vs. soluble versions. This places a high need on effective pharmacological delivery approaches to minimize the loss of activity due to the physical form of the active.

Diiodomethyl-p-tolylsulfone has surprisingly been found to retain 100% of its activity in a dispersed particle form vs. that dissolved in an effective solvent. This activity is presented in the data results in Table 1 below, wherein diiodomethyl-p-tolylsulfone (commercially available as AMICAL 48) is investigated. Particulate materials are desirable so long as biological activity can be maintained, because physical delivery of the active is more efficient and the deposited material can serve as a reservoir of activity over time.

Table 1

| Commercial AMICAL 48 | MIC (ppm)[1] | Retained Activity (%)* |
|---|---|---|
| Dissolved in DMSO | 2.0 | -- |

(continued)

| Commercial AMICAL 48 | MIC (ppm)[1] | Retained Activity (%)* |
|---|---|---|
| Dispersed in Water | 2.0 | 100 |

AMICAL 48, available from Dow

$$* \text{Retained Activity} = \frac{MIC\ in\ DMSO}{MIC\ Dispersed} \times 100$$

In a preferred embodiment of the present invention, the composition comprises at least about 30% retained activity achieved as the diiodomethyl-p-tolylsulfone is present as a particulate dispersion in a liquid solvent, preferably at least about 50% retained activity achieved as the diiodomethyl-p-tolylsulfone is present as a particulate dispersion in a liquid solvent, more preferably at least about 70% retained activity achieved as the diiodomethyl-p-tolylsulfone is present as a particulate dispersion in a liquid solvent.

[1] The Minimum Inhibitory Concentration is indicative of anti-fungal efficacy. Generally, the lower the value of the composition, the better its anti-fungal efficacy, due to increased inherent ability of the anti-dandruff agent to inhibit the growth of microorganisms.

*Malassezia furfur* was grown in a flask containing mDixon medium (see E. Gueho, et al. Antoinie Leeuwenhoek (1996), no. 69, 337-55, which description is incorporated by reference herein). Dilutions of solubilized anti-microbial active were then added to test tubes containing molten mDixon agar. *M. furfur* inoculum was added to each tube of molten agar, the tube vortexed, and the contents poured into separate sterile petri dishes. After the plates are incubated, they were observed for visible *M. furfur* growth. The lowest tested dilution of anti-microbial active that yields no growth is defined as the Minimal Inhibitory Concentration (MIC).

Equipment/Reagents

| | |
|---|---|
| Microbe | *Malassezia furfur* (ATCC 14521) |
| Erlenmeyer flask | 250ml |
| Agar medium | 9.5ml mDixon agar per concentration per active tested |
| Solvent | water, dimethyl sulfonyl oxide ("DMSO") Zinc pyridinethione ZPT having an average particle size of about 2.5μm. |
| Test tubes | 2 tubes per anti-microbial active per concentration per active tested, sterilized, size = 18mm x 150mm |
| Petri dishes | 2 dishes per anti-microbial active per concentration per active tested, sterilized, size = 15mm x 100mm |

Experimental procedure

1) *Malassezia furfur* was grown in a 125 ml Erlenmeyer flask containing 50 ml "mDIXON" medium at 200 rpm and 30°C until turbid.
2) Selected dilutions were prepared using an appropriate dilution series, of the antimicrobial active or combination in solvent, which allowed the sample active to be solubilized prior to addition to the final test agar. For each concentration of the ZPT samples, the solvent was "DMSO"; for other samples, the solvent was water or "DMSO" or other suitable solvent.
3) 0.25 ml dilutions of anti-microbial active were added to test tubes containing 9.5 ml molten "mDIXON" agar (held at 45°C in a water bath).
4) 0.25 *M. furfur* inoculum (adjusted to 5 x $10^5$ cfu/ml by direct count) was added to each test tube of molten agar.
5) Each tube was vortexed, and the contents poured into separate petri dishes.
6) After the agar solidified, the plates inverted and incubated at 30° for 4-5 days.
7) The plates were then observed for visible *M furfur* growth.

PARTICLE SIZE DETERMINATION METHOD

Particle size analyses on diiodomethyl-p-tolylsulfone may be done using the Horiba LA-910 Particle Size Analyzer. The Horiba LA-910 instrument uses the principles of low-angle Fraunhofer Diffraction and Light Scattering to measure the particle size and distribution in a dilute solution of particles. Samples of these two types of raw materials are predispersed in a dilute solution of Lauryl Polyether Alcohol and mixed before introduction to the instrument. On introduction the sample is further diluted and allowed to circulate in the instrument before a measurement is taken. After measurement a calculation algorithm is used to process the data that results in both a particle size and distribution. D (50) is the median particle size or the particle size which corresponds to 50% of the amount of particles are below this size. D(90) is the particle size which corresponds to 90% of the amount of particles are below this size. D(10) is the particle size which corresponds to 10% of the amount of particles are below this size.

J. Methods of Use

The compositions of the present invention may be used in direct application to the skin or in a conventional manner for cleansing skin and hair and controlling microbial infection (including fungal, viral, or bacterial infections) on the skin or scalp. The compositions herein are useful for cleansing the hair and scalp, and other areas of the body such as underarm, feet, and groin areas and for any other area of skin in need of treatment. The present invention may be used for treating or cleansing of the skin or hair of animals as well. An effective amount of the composition, typically from about 1g to about 50g, preferably from about 1g to about 20g of the composition, for cleansing hair, skin or other area of the body, is topically applied to the hair, skin or other area that has preferably been wetted, generally with water, and then rinsed off. Application to the hair typically includes working the shampoo composition through the hair.

A preferred method for providing anti-microbial or anti-fungal (especially antidandruff) efficacy with a shampoo embodiment comprises the steps of: (a) wetting the hair with water, (b) applying an effective amount of the anti-microbial shampoo composition to the hair, and (c) rinsing the anti-microbial shampoo composition from the hair using water. These steps may be repeated as many times as desired to achieve the cleansing, conditioning, and anti-microbial/anti-fungal/anti-dandruff benefits sought.

It is also contemplated that when the anti-microbial active employed is zinc pyrithione, and/or if other optional hair growth regulating agents are employed, the antimicrobial compositions of the present invention, may, provide for the regulation of growth of the hair. The method of regularly using such shampoo compositions comprises repeating steps a, b, and c (above). Further, diiodomethyl-p-tolylsulfone may provide for the regulation of growth of the hair as well.

A further embodiment of the present invention comprises a method comprising the steps of (a) wetting the hair with water, (b) applying an effective amount of a shampoo composition comprising diiodomethyl-p-tolylsulfone, (c) rinsing the shampoo compositions from the hair using water; (d) applying an effective amount of a conditioner composition comprising a diiodomethyl-p-tolylsulfone according to the present invention; (e) rinsing the conditioner composition from the hair using water. A further preferred embodiment of the above mentioned method includes a shampoo composition comprising zinc pyrithione and a conditioner composition comprising diiodomethyl-p-tolylsulfone .

A further embodiment of the present invention comprises a method of treating athlete's foot comprising the use of the composition according to the present invention, a method of treating microbial infections comprising the use of composition as described herein, method of improving the appearance of a scalp comprising the use of the composition according present invention, a method of treating fungal infections comprising the use of the composition according to the present invention, a method of treating topical fungally-mediated conditions, a method of treating dandruff comprising the use of the composition of the present invention, a method of treating diaper dermatitis and candidiasis comprising the use of the compositions of the present invention as described herein, a method of treating tinea capitis comprising the use of the composition according to the present invention, a method of treating yeast infections comprising the use of the composition according to the present invention, a method of treating onychomycosis comprising the use of the composition according to the present invention, and a method of treating underarm body odor.

K. Examples

The following examples further describe and demonstrate the preferred embodiments within the scope of the present invention. The examples are given solely for the purpose of illustration, and are not to be construed as limitations of the present invention since many variations thereof are possible without departing from its scope.

The composition of the invention can be made by mixing one or more selected metal ion sources and one or more metal salts of pyrithione in an appropriate media or carrier, or by adding the individual components separately to the skin or hair cleansing compositions. Useful carriers are discussed more fully above.

1. Topical Compositions

All exemplified compositions can be prepared by conventional formulation and mixing techniques. Component amounts are listed as weight percents and exclude minor materials such as diluents, filler, and so forth. The listed formulations, therefore, comprise the listed components and any minor materials associated with such components. As used herein, "minors" refers to those optional components such as preservatives, viscosity modifiers, pH modifiers, fragrances, foam boosters, and the like. As is apparent to one of ordinary skill in the art, the selection of these minors will vary depending on the physical and chemical characteristics of the particular ingredients selected to make the present invention as described herein. Other modifications can be undertaken by the skilled artisan without departing from the spirit and scope of this invention. These exemplified embodiments of the anti-microbial shampoo, antimicrobial conditioner, and anti-microbial leave-on tonic and deodorants of the present invention may provide excellent anti-microbial efficacy.

L. Methods of Manufacture For Shampoo Compositions

The compositions of the present invention may be prepared by any known or otherwise effective technique, suitable for providing an anti-microbial composition provided that the resulting composition provides the excellent anti-microbial benefits described herein. Methods for preparing the anti-dandruff and conditioning shampoo embodiments of the present invention include conventional formulation and mixing techniques. A method such as that described in U.S. Pat. No. 5,837,661, may be employed, wherein the anti-microbial agent, anti-fungal agent, or anti-dandruff active of the present invention may typically be added in the same step as the silicone premix is added in the U.S. Pat. No. 5,837,661 description.

Antimicrobial Shampoo -Examples 1- 22

A suitable method for preparing the anti-microbial shampoo compositions in Examples 1-22 (below) follows:

About one-third to all of the sodium laureth sulfate (added as 29wt% solution) and acid are added to a jacketed mix tank and heated to about 60°C to about 80°C with slow agitation to form a surfactant solution. The pH of this solution is about 3 to about 7. Sodium benzoate, Cocoamide MEA and fatty alcohols, (where applicable), are added to the tank and allowed to disperse. Ethylene glycol distearate ("EGDS") is added to the mixing vessel and allowed to melt (where applicable). After the EGDS is melted and dispersed, Kathon CG is added to the surfactant solution. The resulting mixture is cooled to about 25°C to about 40°C and collected in a finishing tank. As a result of this cooling step, the EGDS crystallizes to form a crystalline network in the product (where applicable). The remainder of the sodium laureth sulfate and other components, including the silicone and anti-microbial/anti-fungal/anti-dandruff agent(s), are added to the finishing tank with agitation to ensure a homogeneous mixture. Polymers (cationic or nonionic) are dispersed in water or oils as an about 0.1% to about 10% dispersion and/or solution and can be added to the main mix, final mix, or both. Diiodomethyl-p-tolylsulfone can be added to a premix of surfactants or water with or without the aid of a dispersing agent via conventional powder incorporation and mixing techniques into the final mix. Once all components have been added, additional viscosity modifiers, such as sodium chloride and/or sodium xylenesulfonate may be added, as needed, to adjust product viscosity to the extent desired. Product pH may be adjusted, using an acid such as hydrochloric acid, to an acceptable value. Shampoo Compositions - Examples 1-22

| Components | Example 1 | Example 2 | Example 3 | Example 4 | Example 5 | Example 6 | Example 7 | Example 8 | Example 9 |
|---|---|---|---|---|---|---|---|---|---|
| Sodium Laureth Sulfate | 10.00 | 10.0 0 | 10.00 | 10.00 | 10.00 | 10.00 | 10.00 | 10.00 | 10.00 |
| Sodium Lauryl Sulfate | 6.00 | 6.00 | 6.00 | 6.00 | 6.00 | 6.00 | 6.00 | 6.00 | 6.00 |
| Cocamidopropyl Betaine | | | | | | | | | |
| EGDS | 1.50 | 1.50 | 1.50 | 1.50 | 1.50 | 1.50 | 1.50 | 1.50 | 1.50 |
| CMEA | 1.600 | 1.600 | 1.600 | 1.600 | 1.600 | 1.600 | 1.600 | 1.600 | 1.600 |
| Cetyl Alcohol | 0.600 | 0.600 | 0.600 | 0.600 | 0.600 | 0.600 | 0.600 | 0.600 | 0.600 |
| Guar Hydroxy Propyl Trimonium Chloride (1) | 0.500 | 0.500 | 0.500 | 0.500 | 0.500 | 0.500 | 0.500 | 0.500 | 0.500 |
| Polyquaterium-10 (2) | | | | | | | | | |
| Polyquaterium-10 (3) | | | | | | | | | |
| PEG-7M (4) | | | | | | | | | |
| PEG-14M (5) | | | | | | | | | |
| PEG-23M (6) | | | | | | | | | |
| PEG-45M (7) | | | | | | | | | |
| Polyether-1 (8) | | | | | | | | | |
| Dimethicone (9) | 0.85 | 0.85 | 0.85 | 0.85 | 0.85 | 0.85 | 0.85 | 0.85 | 1.00 |
| Diiodomethyl-p-tolylsulfone (10) | 1.000 | 0.500 | 0.250 | 0.125 | 1.250 | 1.500 | 1.750 | 2.000 | 1.000 |
| Hydrochloric Acid (11) | 0.42 | 0.42 | 0.42 | 0.42 | 0.42 | 0.42 | 0.42 | 0.42 | 0.42 |
| Magnesium Sulfate | 0.28 | 0.28 | 0.28 | 0.28 | 0.28 | 0.28 | 0.28 | 0.28 | 0.28 |
| Sodium Chloride | 0.800 | 0.800 | 0.800 | 0.800 | 0.800 | 0.800 | 0.800 | 0.800 | 0.800 |
| Sodium Xylenesulfonate | | | | | | | | | |
| Perfume | 0.750 | 0.750 | 0.750 | 0.750 | 0.750 | 0.750 | 0.750 | 0.750 | 0.750 |
| Sodium Benzoate | 0.250 | 0.250 | 0.250 | 0.250 | 0.250 | 0.250 | 0.250 | 0.250 | 0.250 |
| Kathon | 0.0008 | 0.0008 | 0.0008 | 0.0008 | 0.0008 | 0.0008 | 0.0008 | 0.0008 | 0.0008 |
| Benzyl Alcohol | 0.0225 | 0.0225 | 0.0225 | 0.0225 | 0.0225 | 0.0225 | 0.0225 | 0.0225 | 0.0225 |

(continued)

| Components | Example 1 | Example 2 | Example 3 | Example 4 | Example 5 | Example 6 | Example 7 | Example 8 | Example 9 |
|---|---|---|---|---|---|---|---|---|---|
| Water | Q.S. | Q.S. | Q.S. | Q.S. | Q.S. | Q.S. | Q.S. | Q.S. | Q.S. |

**(1)** Guar having a molecular weight of about 400,000, and having a charge density of about 0.84 meq/g, available from Aqualon.
**(2)** UCARE Polymer JR30M, available from Amerchol
**(3)** UCARE Polymer LR400, available from Amerchol
**(4)** Polyox WSR N-750, available from Amerchol
**(5)** Polyox WSR N-3000, available from Amerchol
**(6)** Polyox WSR N-12K, available from Amerchol
**(7)** Polyox WSR N-60K, available from Amerchol
**(8)** Pure Thix HH, available from Sud Chemie
**(9)** Viscasil 330M available from General Electric Silicones
**(10)** Amical 48, available from Dow
**(11)** 6N HCl, available from J.T. Baker, adjustable to achieve target pH

| Components | Example 10 | Example 11 | Example 12 | Example 13 | Example 14 | Example 15 | Example 16 | Example 17 | Example 18 |
|---|---|---|---|---|---|---|---|---|---|
| Sodium Laureth Sulfate | 10.00 | 10.00 | 10.00 | 10.00 | 10.00 | 10.00 | 10.00 | 12.00 | 14.00 |
| Sodium Lauryl Sulfate | 6.00 | 6.00 | 6.00 | 6.00 | 6.00 | 6.00 | 6.00 | 4.00 | 2.00 |
| Cocamidopropyl Betaine | | | | | | | | | |
| EGDS | 1.50 | 1.50 | 1.50 | 1.50 | 1.50 | 1.50 | 1.50 | 1.50 | 1.50 |
| CMEA | 1.600 | 1.600 | 1.600 | 1.600 | 1.600 | 1.600 | 1.600 | 1.600 | 1.600 |
| Cetyl Alcohol | 0.600 | 0.600 | 0.600 | 0.600 | 0.600 | 0.600 | 0.600 | 0.600 | 0.600 |
| Guar Hydroxy Propyl Trimonium Chloride (1) | 0.500 | 0.500 | 0.500 | 0.500 | 0.500 | | | | |
| Polyquaterium-10 (2) | | | | | | 0.500 | | 0.500 | 0.500 |
| Polyquaterium-10 (3) | | | | | | | 0.500 | | |
| PEG-7M (4) | | | | | | | | | |
| PEG-14M (5) | | | | | | | | | |
| PEG-23M (6) | | | | | | | | | |
| PEG-45M (7) | | | | | | | | | |
| Polyether-1 (8) | | | | | | | | | |
| Dimethicone (9) | 1.35 | 1.60 | 0.85 | 0.85 | 0.85 | 0.85 | 0.85 | 0.85 | 0.85 |
| Diiodomethyl-p-tolylsulfone (10) | 1.000 | 1.000 | 1.000 | 1.000 | 1.000 | 1.000 | 1.000 | 1.000 | 1.000 |
| Hydrochloric Acid (11) | 0.42 | 0.42 | 0.42 | 0.42 | 0.42 | 0.42 | 0.42 | 0.42 | 0.42 |
| Magnesium Sulfate | 0.28 | 0.28 | 0.28 | 0.28 | 0.28 | 0.28 | 0.28 | 0.28 | 0.28 |
| Sodium Chloride | 0.800 | 0.800 | 0.800 | 0.800 | 0.800 | 0.800 | 0.800 | 0.800 | 0.800 |
| Sodium Xylenesulfonate | | | | | | | | | |
| Perfume | 0.750 | 0.750 | 0.350 | 0.600 | 1.000 | 0.750 | 0.750 | 0.750 | 0.750 |
| Sodium Benzoate | 0.250 | 0.250 | 0.250 | 0.250 | 0.250 | 0.250 | 0.250 | 0.250 | 0.250 |
| Kathon | 0.0008 | 0.0008 | 0.0008 | 0.0008 | 0.0008 | 0.0008 | 0.0008 | 0.0008 | 0.0008 |
| Benzyl Alcohol | 0.0225 | 0.0225 | 0.0225 | 0.0225 | 0.0225 | 0.0225 | 0.0225 | 0.0225 | 0.0225 |

(continued)

| Components | Example 10 | Example 11 | Example 12 | Example 13 | Example 14 | Example 15 | Example 16 | Example 17 | Example 18 |
|---|---|---|---|---|---|---|---|---|---|
| Water | Q.S. | Q.S. | Q.S. | Q.S. | Q.S. | Q.S. | Q.S. | Q.S. | Q.S. |

**(1)** Guar having a molecular weight of about 400,000, and having a charge density of about 0.84 meq/g, available from Aqualon.
**(2)** UCARE Polymer JR30M, available from Amerchol
**(3)** UCARE Polymer LR400, available from Amerchol
**(4)** Polyox WSR N-750, available from Amerchol
**(5)** Polyox WSR N-3000, available from Amerchol
**(6)** Polyox WSR N-12K, available from Amerchol
**(7)** Polyox WSR N-60K, available from Amerchol
**(8)** Pure Thix HH, available from Sud Chemie
**(9)** Viscasil 330M available from General Electric Silicones
**(10)** Amical 48, available from Dow
**(11)** 6N HCl, available from J.T. Baker, adjustable to achieve target pH

| Components | Example 19 | Example 20 | Example 21 | Example 22 |
|---|---|---|---|---|
| Sodium Laureth Sulfate | 12.00 | 12.00 | 12.00 | 12.00 |
| Sodium Lauryl Sulfate | 4.00 | 4.00 | 4.00 | 4.00 |
| Cocamidopropyl Betaine | | | | |
| EGDS | 1.50 | 1.50 | 1.50 | 1.50 |
| CMEA | 1.600 | 1.600 | 1.600 | 1.600 |
| Cetyl Alcohol | 0.600 | 0.600 | 0.600 | 0.600 |
| Guar Hydroxy Propyl Trimonium Chloride (1) | | | | |
| Polyquaterium-10 (2) | 0.500 | 0.500 | 0.500 | 0.500 |
| Polyquaterium-10 (3) | | | | |
| PEG-7M (4) | | | | |
| PEG-14M (5) | 0.100 | | | |
| PEG-23M (6) | | 0.100 | | |
| PEG-45M (7) | | | 0.100 | |
| Polyether-1 (8) | | | | 0.100 |
| Dimethicone (9) | 0.85 | 0.85 | 0.85 | 0.85 |
| Diiodomethyl-p-tolylsulfone (10) | 1.000 | 1.000 | 1.000 | 1.000 |
| Hydrochloric Acid (11) | 0.42 | 0.42 | 0.42 | 0.42 |
| Magnesium Sulfate | 0.28 | 0.28 | 0.28 | 0.28 |
| Sodium Chloride | 0.800 | 0.800 | 0.800 | 0.800 |
| Sodium Xylenesulfonate | | | | |
| Perfume | 0.750 | 0.750 | 0.750 | 0.750 |
| Sodium Benzoate | 0.250 | 0.250 | 0.250 | 0.250 |
| Kathon | 0.0008 | 0.0008 | 0.0008 | 0.0008 |
| Benzyl Alcohol | 0.0225 | 0.0225 | 0.0225 | 0.0225 |
| Water | Q.S. | Q.S. | Q.S. | Q.S. |

**(1)** Guar having a molecular weight of about 400,000, and having a charge density of about 0.84 meq/g, available from Aqualon.
**(2)** UCARE Polymer JR30M, available from Amerchol
**(3)** UCARE Polymer LR400, available from Amerchol
**(4)** Polyox WSR N-750, available from Amerchol
**(5)** Polyox WSR N-3000, available from Amerchol
**(6)** Polyox WSR N-12K, available from Amerchol
**(7)** Polyox WSR N-60K, available from Amerchol
**(8)** Pure Thix HH, available from Sud Chemie
**(9)** Viscasil 330M available from General Electric Silicones
**(10)** Amical 48, available from Dow
**(11)** 6N HCl, available from J.T. Baker, adjustable to achieve target pH

| Components | Example 1 | Example 2 | Example 3 | Example 4 | Example 5 | Example 6 | Example 7 | Example 8 | Example 9 |
|---|---|---|---|---|---|---|---|---|---|
| Sodium Laureth Sulfate | 10.00 | 10.00 | 10.00 | 10.00 | 10.00 | 10.00 | 10.00 | 10.00 | 10.00 |
| Sodium Lauryl Sulfate | 6.00 | 6.00 | 6.00 | 6.00 | 6.00 | 6.00 | 6.00 | 6.00 | 6.00 |
| Cocamidopropyl Betaine | | | | | | | | | |
| EGDS | 1.50 | 1.50 | 1.50 | 1.50 | 1.50 | 1.50 | 1.50 | 1.50 | 1.50 |
| CMEA | 1.600 | 1.600 | 1.600 | 1.600 | 1.600 | 1.600 | 1.600 | 1.600 | 1.600 |
| Cetyl Alcohol | 0.600 | 0.600 | 0.600 | 0.600 | 0.600 | 0.600 | 0.600 | 0.600 | 0.600 |
| Guar Hydroxy Propyl Trimonium Chloride (1) | 0.500 | 0.500 | 0.500 | 0.500 | 0.500 | 0.500 | 0.500 | 0.500 | 0.500 |
| Polyquaterium-10 (2) | | | | | | | | | |
| Polyquaterium-10 (3) | | | | | | | | | |
| PEG-7M (4) | | | | | | | | | |
| PEG-14M (5) | | | | | | | | | |
| PEG-23M (6) | | | | | | | | | |
| PEG-45M (7) | | | | | | | | | |
| Polyether-1 (8) | | | | | | | | | |
| Dimethicone (9) | 0.85 | 0.85 | 0.85 | 0.85 | 0.85 | 0.85 | 0.85 | 0.85 | 1.00 |
| Diiodomethyl-p-tolylsulfone (10) | 1.000 | 0.500 | 0.250 | 0.125 | 1.250 | 1.500 | 1.750 | 2.000 | 1.000 |
| Hydrochloric Acid (11) | 0.42 | 0.42 | 0.42 | 0.42 | 0.42 | 0.42 | 0.42 | 0.42 | 0.42 |
| Magnesium Sulfate | 0.28 | 0.28 | 0.28 | 0.28 | 0.28 | 0.28 | 0.28 | 0.28 | 0.28 |
| Sodium Chloride | 0.800 | 0.800 | 0.800 | 0.800 | 0.800 | 0.800 | 0.800 | 0.800 | 0.800 |
| Sodium Xylenesulfonate | | | | | | | | | |
| Perfume | 0.750 | 0.750 | 0.750 | 0.750 | 0.750 | 0.750 | 0.750 | 0.750 | 0.750 |
| Sodium Benzoate | 0.250 | 0.250 | 0.250 | 0.250 | 0.250 | 0.250 | 0.250 | 0.250 | 0.250 |
| Kathon | 0.0008 | 0.0008 | 0.0008 | 0.0008 | 0.0008 | 0.0008 | 0.0008 | 0.0008 | 0.0008 |
| Benzyl Alcohol | 0.0225 | 0.0225 | 0.0225 | 0.0225 | 0.0225 | 0.0225 | 0.0225 | 0.0225 | 0.0225 |

(continued)

| Components | Example 1 | Example 2 | Example 3 | Example 4 | Example 5 | Example 6 | Example 7 | Example 8 | Example 9 |
|---|---|---|---|---|---|---|---|---|---|
| Water | Q.S. | Q.S. | Q.S. | Q.S. | Q.S. | Q.S. | Q.S. | Q.S. | Q.S. |

1)Guar having a molecular weight of about 400,000, and having a charge density of about 0.84 meq/g, available from Aqualon.
2)UCARE Polymer JR30M, available from Amerchol
3)UCARE Polymer LR400, available from Amerchol
4)Polyox WSR N-750, available from Amerchol
5)Polyox WSR N-3000, available from Amerchol
6)Polyox WSR N-12K, available from Amerchol
7)Polyox WSR N-60K, available from Amerchol
8)Pure Thix HH, available from Sud Chemie
9)Viscasil 330M available from General Electric Silicones
10)AMICAL 48, available from Dow
11)6N HCl, available from J.T. Baker, adjustable to achieve target pH

| Components | Example 10 | Example 11 | Example 12 | Example 13 | Example 14 | Example 15 | Example 16 | Example 17 | Example 18 |
|---|---|---|---|---|---|---|---|---|---|
| Sodium Laureth Sulfate | 10.00 | 10.00 | 10.00 | 10.00 | 10.00 | 10.00 | 10.00 | 12.00 | 14.00 |
| Sodium Lauryl Sulfate | 6.00 | 6.00 | 6.00 | 6.00 | 6.00 | 6.00 | 6.00 | 4.00 | 2.00 |
| Cocamidopropyl Betaine | | | | | | | | | |
| EGDS | 1.50 | 1.50 | 1.50 | 1.50 | 1.50 | 1.50 | 1.50 | 1.50 | 1.50 |
| CMEA | 1.600 | 1.600 | 1.600 | 1.600 | 1.600 | 1.600 | 1.600 | 1.600 | 1.600 |
| Cetyl Alcohol | 0.600 | 0.600 | 0.600 | 0.600 | 0.600 | 0.600 | 0.600 | 0.600 | 0.600 |
| Guar Hydroxy Propyl Trimonium Chloride (1) | 0.500 | 0.500 | 0.500 | 0.500 | 0.500 | | | | |
| Polyquaterium-10 (2) | | | | | | 0.500 | | 0.500 | 0.500 |
| Polyquaterium-10 (3) | | | | | | | 0.500 | | |
| PEG-7M (4) | | | | | | | | | |
| PEG-14M (5) | | | | | | | | | |
| PEG-23M (6) | | | | | | | | | |
| PEG-45M (7) | | | | | | | | | |
| Polyether-1 (8) | | | | | | | | | |
| Dimethicone (9) | 1.35 | 1.60 | 0.85 | 0.85 | 0.85 | 0.85 | 0.85 | 0.85 | 0.85 |
| Diiodomethyl-p-tolylsulfone (10) | 1.000 | 1.000 | 1.000 | 1.000 | 1.000 | 1.000 | 1.000 | 1.000 | 1.000 |
| Hydrochloric Acid (11) | 0.42 | 0.42 | 0.42 | 0.42 | 0.42 | 0.42 | 0.42 | 0.42 | 0.42 |
| Magnesium Sulfate | 0.28 | 0.28 | 0.28 | 0.28 | 0.28 | 0.28 | 0.28 | 0.28 | 0.28 |
| Sodium Chloride | 0.800 | 0.800 | 0.800 | 0.800 | 0.800 | 0.800 | 0.800 | 0.800 | 0.800 |
| Sodium Xylenesulfonate | | | | | | | | | |
| Perfume | 0.750 | 0.750 | 0.350 | 0.600 | 1.000 | 0.750 | 0.750 | 0.750 | 0.750 |
| Sodium Benzoate | 0.250 | 0.250 | 0.250 | 0.250 | 0.250 | 0.250 | 0.250 | 0.250 | 0.250 |
| Kathon | 0.0008 | 0.0008 | 0.0008 | 0.0008 | 0.0008 | 0.0008 | 0.0008 | 0.0008 | 0.0008 |
| Benzyl Alcohol | 0.0225 | 0.0225 | 0.0225 | 0.0225 | 0.0225 | 0.0225 | 0.0225 | 0.0225 | 0.0225 |

(continued)

| Components | Example 10 | Example 11 | Example 12 | Example 13 | Example 14 | Example 15 | Example 16 | Example 17 | Example 18 |
|---|---|---|---|---|---|---|---|---|---|
| Water | Q.S. | Q.S. | Q.S. | Q.S. | Q.S. | Q.S. | Q.S. | Q.S. | Q.S. |

1)Guar having a molecular weight of about 400,000, and having a charge density of about 0.84 meq/g, available from Aqualon.
2)UCARE Polymer JR30M, available from Amerchol
3)UCARE Polymer LR400, available from Amerchol
4)Polyox WSR N-750, available from Amerchol
5)Polyox WSR N-3000, available from Amerchol
6)Polyox WSR N-12K, available from Amerchol
7)Polyox WSR N-60K, available from Amerchol
8)Pure Thix HH, available from Sud Chemie
9)Viscasil 330M available from General Electric Silicones
10)AMICAL 48, available from Dow
11)6N HCl, available from J.T. Baker, adjustable to achieve target pH

| Components | Example 19 | Example 20 | Example 21 | Example 22 |
|---|---|---|---|---|
| Sodium Laureth Sulfate | 12.00 | 12.00 | 12.00 | 12.00 |
| Sodium Lauryl Sulfate | 4.00 | 4.00 | 4.00 | 4.00 |
| Cocamidopropyl Betaine | | | | |
| EGDS | 1.50 | 1.50 | 1.50 | 1.50 |
| CMEA | 1.600 | 1.600 | 1.600 | 1.600 |
| Cetyl Alcohol | 0.600 | 0.600 | 0.600 | 0.600 |
| Guar Hydroxy Propyl Trimonium Chloride (1) | | | | |
| Polyquaterium-10 (2) | 0.500 | 0.500 | 0.500 | 0.500 |
| Polyquaterium-10 (3) | | | | |
| PEG-7M (4) | | | | |
| PEG-14M (5) | 0.100 | | | |
| PEG-23M (6) | | 0.100 | | |
| PEG-45M (7) | | | 0.100 | |
| Polyether-1 (8) | | | | 0.100 |
| Dimethicone (9) | 0.85 | 0.85 | 0.85 | 0.85 |
| Diiodomethyl-p-tolylsulfone (10) | 1.000 | 1.000 | 1.000 | 1.000 |
| Hydrochloric Acid (11) | 0.42 | 0.42 | 0.42 | 0.42 |
| Magnesium Sulfate | 0.28 | 0.28 | 0.28 | 0.28 |
| Sodium Chloride | 0.800 | 0.800 | 0.800 | 0.800 |
| Sodium Xylenesulfonate | | | | |
| Perfume | 0.750 | 0.750 | 0.750 | 0.750 |
| Sodium Benzoate | 0.250 | 0.250 | 0.250 | 0.250 |
| Kathon | 0.0008 | 0.0008 | 0.0008 | 0.0008 |
| Benzyl Alcohol | 0.0225 | 0.0225 | 0.0225 | 0.0225 |
| Water | Q.S. | Q.S. | Q.S. | Q.S. |

1)Guar having a molecular weight of about 400,000, and having a charge density of about 0.84 meq/g, available from Aqualon.
2)UCARE Polymer JR30M, available from Amerchol
3)UCARE Polymer LR400, available from Amerchol
4)Polyox WSR N-750, available from Amerchol
5)Polyox WSR N-3000, available from Amerchol
6)Polyox WSR N-12K, available from Amerchol
7)Polyox WSR N-60K, available from Amerchol
8)Pure Thix HH, available from Sud Chemie
9)Viscasil 330M available from General Electric Silicones
10)AMICAL 48, available from Dow
11)6N HCl, available from J.T. Baker, adjustable to achieve target pH

Cleansing Compositions - Examples 23-31

A suitable method for preparing the anti-microbial cleansing compositions in Examples 23-31 (below) follows:

Components 1-3, 6, and 7 are mixed with heating to 190F. Components 4, 9, 12, and 14 are mixed at room temperature in a separate pot. After the first mixture has reached 190F, it is added to the second mixture. After this

mixture has cooled below 140 F, component 10 is added. In a separate vessel at 160 F, the petrolatum and diiodomethyl-p-tolylsulfone are mixed. When the aqueous phase has cooled below 110 F, the petrolatum/diiodomethyl-p-tolylsulfone is added and agitated until smooth. Diiodomethyl-p-tolylsulfone can also be added to a premix of surfactants or water with or without the aid of a dispersing agent via conventional powder incorporation and mixing techniques into the cooled mixture. Finally the perfume is added.

| Components | Example 23 | Example 24 | Example 25 | Example 26 | Example 27 | Example 28 | Example 29 | Example 30 | Example 31 |
|---|---|---|---|---|---|---|---|---|---|
| 1.Sodium Lauryl Sulfate | 4.000 | 4.000 | 4.000 | 4.000 | 4.000 | 4.000 | 4.000 | 4.000 | 4.000 |
| 2.Sodium Laureth Sulfate | 3.000 | 3.000 | 3.000 | 3.000 | 3.000 | 3.000 | 3.000 | 3.000 | 3.000 |
| 3.Sodium Laruroamphoacetate | 4.000 | 4.000 | 4.000 | 4.000 | 4.000 | 4.000 | 4.000 | 4.000 | 4.000 |
| 4.Sodium Lauroyl Sarcosinate | 2.000 | 2.000 | 2.000 | 2.000 | 2.000 | 2.000 | 2.000 | 2.000 | 2.000 |
| 5.Diiodomethyl-p-tolylsulfone (1) | 1.000 | 0.750 | 0.500 | 0.250 | 0.125 | 1.250 | 1.500 | 1.750 | 2.000 |
| 6.Lauric Acid | 1.000 | 1.000 | 1.000 | 1.000 | 1.000 | 1.000 | 1.000 | 1.000 | 1.000 |
| 7.Trihydroxys tearin | 0.650 | 0.650 | 0.650 | 0.650 | 0.650 | 0.650 | 0.650 | 0.650 | 0.650 |
| 8.Citric Acid | 0.750 | 0.750 | 0.750 | 0.750 | 0.750 | 0.750 | 0.750 | 0.750 | 0.750 |
| 9.Sodium Benzoate | 0.250 | 0.250 | 0.250 | 0.250 | 0.250 | 0.250 | 0.250 | 0.250 | 0.250 |
| 10.Glydant | 0.120 | 0.120 | 0.120 | 0.120 | 0.120 | 0.120 | 0.120 | 0.120 | 0.120 |
| 11.Perfume | 0.750 | 0.750 | 0.750 | 0.750 | 0.750 | 0.750 | 0.750 | 0.750 | 0.750 |
| 12.Polyquaterium-10 (2) | 0.750 | 0.750 | 0.750 | 0.750 | 0.750 | 0.750 | 0.750 | 0.750 | 0.750 |
| 13.Petrolatum | 15.000 | 15.000 | 15.000 | 15.000 | 15.000 | 15.000 | 15.000 | 15.000 | 15.000 |
| 14.Water | Q.S. | Q.S. | Q.S. | Q.S. | Q.S. | Q.S. | Q.S. | Q.S. | Q.S. |
| (1) AMICAL 48, available from Dow<br>(2) UCARE Polymer JR30M, available from Amerchol | | | | | | | | | |

Cleansing/ Facial Compositions - Examples 32-49

A suitable method for preparing the anti-microbial cleansing/ facial compositions in Examples 32-49 are known to those skilled in the art, and may be prepared by any known or otherwise effective technique, suitable for providing an anti-microbial cleansing/ facial composition provided that the resulting composition provides the excellent anti-microbial benefits described herein. Methods for preparing the antimicrobial cleansing/ facial compositions embodiments of the present invention may include conventional formulation and mixing techniques. A method such as that described in U.S. Pat. No. 5,665,364, may be employed.

| Components | Example 32 | Example 33 | Example 34 | Example 35 | Example 36 | Example 37 | Example 38 | Example 39 | Example 40 |
|---|---|---|---|---|---|---|---|---|---|
| Cetyl Betaine | 6.667 | 6.667 | 6.667 | 6.667 | 6.667 | 6.667 | 6.667 | 6.667 | 6.667 |
| PPG-15 Stearyl Ether | 4.000 | 4.000 | 4.000 | 4.000 | 4.000 | 4.000 | 4.000 | 4.000 | 4.000 |
| Sodium Lauryl Sulfate | 3.571 | 3.571 | 3.571 | 3.571 | 3.571 | 3.571 | 3.571 | 3.571 | 3.571 |
| Glycerin | 3.000 | 3.000 | 3.000 | 3.000 | 3.000 | 3.000 | 3.000 | 3.000 | 3.000 |
| Stearyl Alcohol | 2.880 | 2.880 | 2.880 | 2.880 | 2.880 | 2.880 | 2.880 | 2.880 | 2.880 |
| Distearyldim onium Chloride | 1.500 | 1.500 | 1.500 | 1.500 | 1.500 | 1.500 | 1.500 | 1.500 | 1.500 |
| Oxidized Polyethylene | 1.000 | 1.000 | 1.000 | 1.000 | 1.000 | 1.000 | 1.000 | 1.000 | 1.000 |
| Diiodomethyl-p-tolylsulfone (1) | 1.000 | 0.750 | 0.50 0 | 0.250 | 0.125 | 1.250 | 1.500 | 1.750 | 2.000 |
| Cetyl Alcohol | 0.800 | 0.800 | 0.800 | 0.800 | 0.800 | 0.800 | 0.800 | 0.800 | 0.800 |
| Steareth-21 | 0.500 | 0.500 | 0.500 | 0.500 | 0.500 | 0.500 | 0.500 | 0.500 | 0.500 |
| Behenyl Alcohol | 0.320 | 0.320 | 0.320 | 0.320 | 0.320 | 0.320 | 0.320 | 0.320 | 0.320 |
| PPG-30 | 0.250 | 0.250 | 0.250 | 0.250 | 0.250 | 0.250 | 0.250 | 0.250 | 0.250 |
| Steareth-2 | 0.250 | 0.250 | 0.250 | 0.250 | 0.250 | 0.250 | 0.250 | 0.250 | 0.250 |
| Perfume | 0.200 | 0.200 | 0.200 | 0.200 | 0.200 | 0.200 | 0.200 | 0.200 | 0.200 |
| Citric Acid | As Needed | As Needed | As Needed | As Needed | As Needed | As Needed | As Needed | As Needed | As Needed |
| Sodium Citrate | As Needed | As Needed | As Needed | As Needed | As Needed | As Needed | As Needed | As Needed | As Needed |
| Water | Q.S. | Q.S. | Q.S. | Q.S. | Q.S. | Q.S. | Q.S. | Q.S. | Q.S. |
| (1) AMICAL 48, available from Dow | | | | | | | | | |

| Components | Example 41 | Example 42 | Example 43 | Example 44 | Example 45 | Example 46 | Example 47 | Example 48 | Example 49 |
|---|---|---|---|---|---|---|---|---|---|
| Sodium Laureth Sulfate | 8.000 | 8.000 | 8.000 | 8.000 | 8.000 | 8.000 | 8.000 | 8.000 | 8.000 |
| Disodium Cocamphodiacetate | 7.000 | 7.000 | 7.000 | 7.000 | 7.000 | 7.000 | 7.000 | 7.000 | 7.000 |
| PEG-80 Glyceryl Cocoate | 3.500 | 3.500 | 3.500 | 3.500 | 3.500 | 3.500 | 3.500 | 3.500 | 3.500 |
| Sodium Chloride | 2.170 | 2.170 | 2.170 | 2.170 | 2.170 | 2.170 | 2.170 | 2.170 | 2.170 |
| Glycol Distearate | 2.000 | 2.000 | 2.000 | 2.000 | 2.000 | 2.000 | 2.000 | 2.000 | 2.000 |
| Diiodomethyl-p-tolylsulfone (1) | 1.000 | 0.750 | 0.500 | 0.250 | 0.125 | 1.250 | 1.500 | 1.750 | 2.000 |
| Dimethicone | 0.900 | 0.900 | 0.900 | 0.900 | 0.900 | 0.900 | 0.900 | 0.900 | 0.900 |
| Sodium Trideceth-7 Carboxylate | 0.502 | 0.502 | 0.502 | 0.502 | 0.502 | 0.502 | 0.502 | 0.502 | 0.502 |
| Perfume | 0.320 | 0.320 | 0.320 | 0.320 | 0.320 | 0.320 | 0.320 | 0.320 | 0.320 |
| Citric Acid | 0.276 | 0.276 | 0.276 | 0.276 | 0.276 | 0.276 | 0.276 | 0.276 | 0.276 |
| Quatemium-15 | 0.150 | 0.150 | 0.150 | 0.150 | 0.150 | 0.150 | 0.150 | 0.150 | 0.150 |
| Polyquaterium-10 (2) | 0.150 | 0.150 | 0.150 | 0.150 | 0.150 | 0.150 | 0.150 | 0.150 | 0.150 |
| PEG-30 Glyceryl Cocoate | As Needed | As Needed | As Needed | As Needed | As Needed | As Needed | As Needed | As Needed | As Needed |
| Water | Q.S. | Q.S. | Q.S. | Q.S. | Q.S. | Q.S. | Q.S. | Q.S. | Q.S. |

(1) AMICAL 48, available from Dow
(2) UCARE Polymer JR30M, available from Amerchol

Hair Conditioning Composition -Examples 50-73

A suitable method for preparing the anti-microbial hair conditioning compositions in Examples 50-73 (below) by conventional formulation and mixing techniques follows:

When included in the composition, polymeric materials such as polypropylene glycol are dispersed in water at room temperature to make a polymer solution, and heated up to above 70°C. Amidoamine and acid, and when present, other cationic surfactants, ester oil of low melting point oil are added in the solution with agitation. Then high melting point fatty compound, and when present, other low melting point oils and benzyl alcohol are also added in the solution with agitation. The mixture thus obtained is cooled down to below 60°C, and the remaining components such as diiodomethyl-p-tolylsulfone and silicone compound are added with agitation, and further cooled down to about 30°C.

A triblender and/or mill can be used in each step, if necessary to disperse the materials. Alternatively, up to 50% of the acid can be added after cooling below 60°C.

The embodiments disclosed herein have many advantages. For example, they may provide effective anti-microbial, especially anti-dandruff, efficacy, while not deteriorating conditioning benefits such as wet hair feel, spreadability, and rinsability, as well as providing glossiness, and dry combing.

| Components | Example 50 | Example 51 | Example 52 | Example 53 | Example 54 | Example 55 | Example 56 | Example 57 | Example 58 |
|---|---|---|---|---|---|---|---|---|---|
| L-Glutamic Acid | 0.640 | 0.640 | 0.640 | 0.640 | 0.640 | 0.412 | 0.412 | 0.412 | 0.412 |
| Stearamidopropyldimethyl amine | 2.000 | 2.000 | 2.000 | 2.000 | 2.000 | 1.600 | 1.600 | 1.600 | 1.600 |
| Behentrimonium Chloride | | | | | | | | | |
| Quaterium-18 | | | | | | | | | |
| Cetyl Alcohol | 2.500 | 2.500 | 2.500 | 2.500 | 2.500 | 2.000 | 2.000 | 2.000 | 2.000 |
| Stearyl Alcohol | 4.500 | 4.500 | 4.500 | 4.500 | 4.500 | 3.600 | 3.600 | 3.600 | 3.600 |
| Cetearyl Alcohol | | | | | | | | | |
| Polysorbate 60 | | | | | | | | | |
| Glyceral Monostearate | | | | | | | | | |
| Oleyl Alcohol | | | | | | | | | |
| Hydroxyethylcellulose | | | | | | | | | |
| Peg 2M (1) | | | | | | | | | |
| Dimethicone (2) | | | | | | 0.200 | 0.200 | 0.200 | 0.200 |
| Dimethicone (3) | 0.630 | 0.630 | 0.630 | 0.630 | 0.630 | | | | |
| Cyclopentasiloxane (3) | 3.570 | 3.570 | 3.570 | 3.570 | 3.570 | | | | |
| Benzyl Alcohol | 0.400 | 0.400 | 0.400 | 0.400 | 0.400 | 0.400 | 0.400 | 0.400 | 0.400 |
| Methyl Paraben | 0.200 | 0.200 | 0.200 | 0.200 | 0.200 | 0.200 | 0.200 | 0.200 | 0.200 |
| Propyl Paraben | 0.100 | 0.100 | 0.100 | 0.100 | 0.100 | 0.100 | 0.100 | 0.100 | 0.100 |
| Phenoxy Ethanol | 0.300 | 0.300 | 0.300 | 0.300 | 0.300 | 0.300 | 0.300 | 0.300 | 0.300 |
| Sodium Chloride | 0.010 | 0.010 | 0.010 | 0.010 | 0.010 | 0.010 | 0.010 | 0.010 | 0.010 |
| Diiodomethyl-p-tolylsulfone (4) | 0.100 | 0.250 | 0.500 | 1.000 | 1.250 | 0.100 | 0.250 | 0.500 | 1.000 |
| Citric Acid | 0.130 | 0.130 | 0.130 | 0.130 | 0.130 | 0.130 | 0.130 | 0.130 | 0.130 |
| Kathon | | | | | | | | | |
| Perfume | 0.400 | 0.400 | 0.400 | 0.40 0 | 0.400 | 0.400 | 0.400 | 0.400 | 0.400 |

(continued)

| Components | Example 50 | Example 51 | Example 52 | Example 53 | Example 54 | Example 55 | Example 56 | Example 57 | Example 58 |
|---|---|---|---|---|---|---|---|---|---|
| Sodium | | | | | | | | | |
| Hydroxide | | | | | | | | | |
| Isopropyl Alcohol | | | | | | | | | |
| Water | Q.S. | Q.S. | Q.S. | Q.S. | Q.S. | Q.S. | Q.S. | Q.S. | Q.S. |

**(1)** Polyox WSR N-10 available from Amerchol Corp.
**(2)** 10,000cps Dimethicone TSF451-1MA available from GE
**(3)** 15/85 Dimethicone/ Cyclomethicone Blend available from GE
**(4)** AMICAL 48, available from Dow

| Components | Example 59 | Example 60 | Example 61 | Example 62 | Example 63 | Example 64 | Example 65 | Example 66 | Example 67 |
|---|---|---|---|---|---|---|---|---|---|
| L-Glutamic Acid | | | | | | | | | |
| Stearamidopropyldimethylamine | 1.000 | 1.000 | 1.000 | 1.000 | 1.000 | 1.000 | 1.000 | 1.000 | |
| Behentrimonium Chloride | | | | | | | | | 3.380 |
| Quaterium-18 | 0.750 | 0.750 | 0.750 | 0.750 | 0.750 | 0.750 | 0.750 | 0.750 | |
| Cetyl Alcohol | 0.960 | 0.960 | 0.960 | 0.960 | 0.960 | 0.960 | 0.960 | 0.960 | 2.320 |
| Stearyl Alcohol | 0.640 | 0.640 | 0.640 | 0.640 | 0.640 | 0.640 | 0.640 | 0.640 | 4.180 |
| Cetearyl Alcohol | 0.500 | 0.500 | 0.500 | 0.500 | 0.500 | 0.500 | 0.500 | 0.500 | |
| Polysorbate 60 | 0.500 | 0.500 | 0.500 | 0.500 | 0.500 | 0.500 | 0.500 | 0.500 | |
| Glyceral Monostearat e | 0.250 | 0.250 | 0.250 | 0.250 | 0.250 | 0.250 | 0.250 | 0.250 | |
| Oleyl Alcohol | 0.250 | 0.250 | 0.250 | 0.250 | 0.250 | 0.250 | 0.250 | 0.250 | |
| Hydroxyethylcellulose | 0.250 | 0.250 | 0.250 | 0.250 | 0.250 | 0.250 | 0.250 | 0.250 | |
| Peg 2M (1) | 0.500 | 0.500 | 0.500 | 0.500 | 0.500 | 0.500 | 0.500 | 0.500 | |
| Dimethicone (2) | | | | | | 0.252 | 0.252 | 0.252 | |
| Dimethicone (3) | 0.630 | 0.630 | 0.630 | 0.630 | 0.630 | | | | 0.630 |
| Cyclopentasiloxane (3) | 3.570 | 3.570 | 3.570 | 3.570 | 3.570 | | | | 3.570 |
| Benzyl Alcohol | 0.400 | 0.400 | 0.400 | 0.400 | 0.400 | 0.400 | 0.400 | 0.400 | 0.400 |
| Methyl Paraben | 0.200 | 0.200 | 0.200 | 0.200 | 0.200 | 0.200 | 0.200 | 0.200 | 0.200 |
| Propyl Paraben | 0.100 | 0.100 | 0.100 | 0.100 | 0.100 | 0.100 | 0.100 | 0.100 | 0.100 |
| Phenoxy Ethanol | 0.300 | 0.300 | 0.300 | 0.300 | 0.300 | 0.300 | 0.300 | 0.300 | 0.300 |
| Sodium Chloride | | | | | | | | | |
| Diiodomethyl-p-tolylsulfone (4) | 1.000 | 0.500 | 0.250 | 0.125 | 1.500 | 1.000 | 0.500 | 0.250 | 0.100 |
| Citric Acid | 0.200 | 0.200 | 0.200 | 0.200 | 0.200 | 0.200 | 0.200 | 0.200 | |
| Kathon | | | | | | | | | |
| Perfume | 0.400 | 0.400 | 0.400 | 0.400 | 0.400 | 0.400 | 0.400 | 0.400 | 0.300 |
| Sodium Hydroxide | | | | | | | | | 0.014 |

(continued)

| Components | Example 59 | Example 60 | Example 61 | Example 62 | Example 63 | Example 64 | Example 65 | Example 66 | Example 67 |
|---|---|---|---|---|---|---|---|---|---|
| Isopropyl Alcohol | | | | | | | | | 0.507 |
| Water | Q.S. | Q.S. | Q.S. | Q.S. | Q.S. | Q.S. | Q.S. | Q.S. | Q.S. |

**(1)** Polyox WSR N-10 available from Amerchol Corp.
**(2)** 10,000cps Dimethicone TSF451-1MA available from GE
**(3)** 15/85 Dimethicone/ Cyclomethicone Blend available from GE
**(4)** AMICAL 48, available from Dow

| Components | Example 68 | Example 69 | Example 70 | Example 71 | Example 72 | Example 73 |
|---|---|---|---|---|---|---|
| L-Glutamic Acid | | | | | | |
| Stearamidopropyldimethylamine | | | | | | |
| Behentrimonium Chloride | 3.380 | 3.380 | 3.380 | 3.380 | 3.380 | 3.380 |
| Quaterium-18 | | | | | | |
| Cetyl Alcohol | 2.320 | 2.320 | 2.320 | 2.320 | 2.320 | 2.320 |
| Stearyl Alcohol | 4.180 | 4.180 | 4.180 | 4.180 | 4.180 | 4.180 |
| Cetearyl Alcohol | | | | | | |
| Polysorbate 60 | | | | | | |
| Glyceral Monostearate | | | | | | |
| Oleyl Alcohol | | | | | | |
| Hydroxyethylcellulose | | | | | | |
| Peg 2M (1) | | | | | | |
| Dimethicone (2) | | | | | | |
| Dimethicone (3) | 0.630 | 0.630 | 0.630 | 0.630 | 0.630 | 0.630 |
| Cyclopentasiloxane (3) | 3.570 | 3.570 | 3.570 | 3.570 | 3.570 | 3.570 |
| Benzyl Alcohol | 0.400 | 0.400 | 0.400 | 0.400 | 0.400 | 0.400 |
| Methyl Paraben | 0.200 | 0.200 | 0.200 | 0.200 | 0.200 | 0.200 |
| Propyl Paraben | 0.100 | 0.100 | 0.100 | 0.100 | 0.100 | 0.100 |
| Phenoxy Ethanol | 0.300 | 0.300 | 0.300 | 0.300 | 0.300 | 0.300 |
| Sodium Chloride | | | | | | |
| Diiodomethyl-p-tolylsulfone (4) | 1.000 | 0.500 | 0.250 | 0.125 | 1.500 | 2.000 |
| Citric Acid | | | | | | |
| Kathon | | | | | | |
| Perfume | 0.300 | 0.300 | 0.300 | 0.300 | 0.300 | 0.300 |
| Sodium Hydroxide | 0.014 | 0.014 | 0.014 | 0.014 | 0.014 | 0.014 |
| Isopropyl Alcohol | 0.507 | 0.507 | 0.507 | 0.507 | 0.507 | 0.507 |
| Water | Q.S. | Q.S. | Q.S. | Q.S. | Q.S. | Q.S. |
| **(1)** Polyox WSR N-10 available from Amerchol Corp.<br>**(2)** 10,000cps Dimethicone TSF451-1MA available from GE<br>**(3)** 15/85 Dimethicone/ Cyclomethicone Blend available from GE<br>**(4)** AMICAL 48, available from Dow | | | | | | |

Anti-Microbial Leave-In Hair Tonic - Examples 74-80

A suitable method for preparing the anti-microbial leave-in hair tonic compositions in Examples 74-80 (below) follows:

Add most of the formula water; with stirring, add carbomer and mix until fully dispersed. In a separate vessel, add ethanol and then molten PEG-60 hydrogenated castor oil and perfume. Transfer this to main mix tank with agitation. Add other water soluble ingredients, minors, and diiodomethyl-p-tolylsulfone. Slowly add styryl silicone and let stir. Add triethanolamine slowly with stirring.

| Components | Example 74 | Example 75 | Example 76 | Example 77 | Exam ple 78 | Example 79 | Example 80 |
|---|---|---|---|---|---|---|---|
| Carbomer | 0.1 | 0.1 | 0.1 | 0.1 | 0.1 | 0.1 | 0.1 |
| Acrylates/C 10 -20 alkyl acrylate crosspolymer | 0.2 | 0.2 | 0.2 | 0.2 | 0.2 | 0.2 | 0.2 |
| Triethanolamine | 0.3 | 0.3 | 0.3 | 0.3 | 0.3 | 0.3 | 0.3 |
| Diiodomethyl-p-tolylsulfone (1) | 0.1 | 0.25 | 0.5 | 0.75 | 1 | 1.25 | 1.5 |
| Camphor | 0.05 | 0.05 | 0.05 | 0.05 | 0.05 | 0.05 | 0.05 |
| Menthol | 0.5 | 0.5 | 0.5 | 0.5 | 0.5 | 0.5 | 0.5 |
| Panthenol | 0.05 | 0.05 | 0.05 | 0.05 | 0.05 | 0.05 | 0.05 |
| Pantyl Ethyl Ether | 0.05 | 0.05 | 0.05 | 0.05 | 0.05 | 0.05 | 0.05 |
| Lactic Acid | 0.05 | 0.05 | 0.05 | 0.05 | 0.05 | 0.05 | 0.05 |
| Styryl Silicone | 0.5 | 0.5 | 0.5 | 0.5 | 0.5 | 0.5 | 0.5 |
| Ceteareth-20 | 0.15 | 0.15 | 0.15 | 0.15 | 0.15 | 0.15 | 0.15 |
| PEG-60 Hydrogenated Castor Oil | 0.15 | 0.15 | 0.15 | 0.15 | 0.15 | 0.15 | 0.15 |
| Perfume | 0.5 | 0.5 | 0.5 | 0.5 | 0.5 | 0.5 | 0.5 |
| Water | Q.S. | Q.S. | Q.S. | Q.S. | Q.S. | Q.S. | Q.S. |
| (1) AMICAL 48, available from Dow | | | | | | | |

Oil-in-Water cream/lotion - Examples 81-85

The oil-in-water cream/lotion compositions of Examples 81-85 may be prepared by any known or otherwise effective conventional method.

| Components | Example 81 | Example 82 | Example 83 | Example 84 | Example 85 |
|---|---|---|---|---|---|
| Oil Phase | | | | | |
| Mineral oil | 15 | 20 | 20 | 20 | 20 |
| Polysorbate | 2 | 3 | 3 | 3 | 3 |
| Aqueous Phase | | | | | |
| Diiodomethyl-p-tolylsulfone (1) | 1 | 1 | 0.5 | 0.25 | 2 |
| Preservative | 0.3 | 0.3 | 0.3 | 0.3 | 0.3 |
| Perfume | 0.2 | 0.2 | 0.2 | 0.2 | 0.2 |
| Water | Q.S. | Q.S. | Q.S. | Q.S. | Q.S. |
| (1) AMICAL 48, available from Dow | | | | | |

Deodorant Composition -Examples 86-90

The deodorant compositions of Examples 86-90 may be prepared by any known or otherwise effective conventional method.

| Components | Example 86 | Example 87 | Example 88 | Example 89 | Example 90 |
|---|---|---|---|---|---|
| Dipropylene glycol | 10 | 10 | 10 | 10 | 10 |
| Tetra-propylene glycol | 45 | 45 | 45 | 45 | 45 |
| Hexylene glycol | 10 | 10 | 10 | 10 | 10 |
| PPG-3 myristyl ether | 1.7 | 1.5 | 1.6 | 1.7 | 1 |
| Triclosan | 0.3 | 0.3 | 0.3 | 0.3 | 0.3 |
| Diiodomethyl-p-tolylsulfone (1) | 1 | 1 | 0.5 | 0.25 | 2 |
| Sodium hydroxide, 50% soln. | 0.04 | 0.04 | 0.04 | 0.04 | 0.04 |
| Tetrasodium EDTA | 0.025 | 0.025 | 0.025 | 0.025 | 0.025 |
| Sodium Stearate | 5.5 | 5.5 | 5.5 | 5.5 | 5.5 |
| Fragrance | 3 | 3 | 4 | 4 | 3 |
| Color | 1.5 | 1.5 | 1.5 | 1.5 | 1.5 |
| Water | 22 | 22 | 22 | 22 | 22 |
| (1) AMICAL 48, available from Dow | | | | | |

10. Other Ingredients

The present invention may, in some embodiments, further comprise additional optional components known or otherwise effective for use in hair care or personal care products. The concentration of such optional ingredients generally ranges from zero to about 25%, more typically from about 0.05% to about 20%, even more typically from about 0.1% to about 15%, by weight of the composition. Such optional components should also be physically and chemically compatible with the essential components described herein, and should not otherwise unduly impair product stability, aesthetics or performance.

Non-limiting examples of optional components for use in the present invention include anti-static agents, foam boosters, anti-dandruff agents in addition to the antidandruff agents described above, viscosity adjusting agents and thickeners, suspension materials (e.g. EGDS, thixins), pH adjusting agents (e.g. sodium citrate, citric acid, succinic acid, sodium succinate, sodium maleate, sodium glycolate, malic acid, glycolic acid, hydrochloric acid, sulfuric acid, sodium bicarbonate, sodium hydroxide, and sodium carbonate), preservatives (e.g. DMDM hydantoin), anti-microbial agents (e.g. triclosan or triclocarbon), dyes, organic solvents or diluents, pearlescent aids, perfumes, fatty alcohols, proteins, skin active agents, sunscreens, vitamins (such as retinoids including retinyl propionate, vitamin E such as tocopherol acetate, panthenol, and vitamin B3 compounds including niacinamide), emulsifiers,volatile carriers, select stability actives, styling polymers, organic styling polymers, silicone-grafted styling polymers, cationic spreading agents, pediculocides, foam boosters, viscosity modifiers and thickeners, polyalkylene glycols and combinations thereof.

Optional anti-static agents such as water-insoluble cationic surfactants may be used, typically in concentrations ranging from about 0.1 % to about 5%, by weight of the composition. Such anti-static agents should not unduly interfere with the in-use performance and end-benefits of the anti-microbial composition; particularly, the antistatic agent should not interfere with the anionic surfactant. A specific non-limiting example of a suitable anti-static agents is tricetyl methyl ammonium chloride.

Optional foam boosters for use in the present invention described herein include fatty ester (e.g. $C_8$-$C_{22}$) mono- and di ($C_1$-$C_5$, especially $C_1$-$C_3$) alkanol amides. Specific non-limiting examples of such foam boosters include coconut monoethanolamide, coconut diethanolamide, and mixtures thereof.

Optional viscosity modifiers and thickeners may be used, typically in amounts effective for the anti-microbial compositions of the present invention to generally have an overall viscosity from about 1,000 csk to about 20,000 csk, preferably from about 3,000 csk to about 10,000 csk. Specific non-limiting examples of such viscosity modifiers and thickeners include: sodium chloride, sodium sulfate, and mixtures thereof.

## Claims

**1.** A composition comprising:

From 12 wt.% to 22 wt.%, based on the total weight of the composition, of anionic detersive surfactant;
From 0.05 wt.% to 3 wt.%, based on the total weight of the composition, of a cationic polymer; and
an effective amount of diiodomethyl-p-tolylsulfone wherein the diiodomethyl-p-tolylsulfone is present as a particulate dispersion in a liquid solvent consisting of water or a mixture of water and water-miscible co-solvent.

**2.** A composition according to claim 1, comprising additional surfactants.

**3.** A composition according to Claim 1, wherein at least 30% retained activity is achieved as the diiodomethyl-p-tolylsulfone is present as a Particulate dispersion in a liquid solvent.

**4.** A composition according to Claim 1, wherein greater than 70 wt.% of the diiodomethyl-p-tolylsulfone remains in the form of a particulate dispersion in a liquid solvent.

**5.** A composition according to Claim 1, wherein diiodomethyl-p-tolylsulfone has a particle size with a $D_{50}$ of less than 30 microns, preferably with a $D_{50}$ of less than 15 microns, more preferably with a $D_{50}$ of less than 7.5 microns.

**6.** A composition according to Claim 1, wherein diiodomethyl-p-tolylsulfone is present from 0.001 wt.% to 10 wt.%, preferably from 0.01 wt.% to 5wt. %, more preferably from 0.1 wt.% to 3 wt.% based on the total weight of the composition.

**7.** A composition according to Claim 1, wherein the pH of the composition is from 2 to 11, preferably from 4 to 9, more preferably from 6 to 8.

**8.** A composition according to Claim 1, wherein the composition further comprises a dispersing aide.

**9.** A composition according to Claim 1, wherein the composition further comprises a suspending agent.

**10.** A composition according to any one of claims 1 to 9, comprising from 0.1 to 1.0 wt.%, based on the total weight of the composition, of the cationic polymer.

**11.** A composition according to any one of claims 1 to 10, further comprising a non-ionic polymer selected from polyalkylene glycols having a molecular weight of more than 1000.

**12.** A composition according to any one of claims 1 to 11, further comprising from 0.001 wt.% to 10 wt.%, based on the total weight of the composition, of an insoluble silicone conditioning agent.

**13.** Use of a composition as defined in any one claims 1 to 12 for the manufacture of a topically applied medicament for the treatment or prevention of microbial infection of the skin or scalp.

**Patentansprüche**

**1.** Zusammensetzung, umfassend:

basierend auf dem Gesamtgewicht der Zusammensetzung zu 12 Gew.-% bis 22 Gew.-% anionisches Reinigungstensid,
basierend auf dem Gesamtgewicht der Zusammensetzung zu 0,05 Gew.-% bis 3 Gew.-% ein kationisches Polymer und
eine wirksame Menge von Diiodomethyl-p-tolylsulfon, wobei das Diiodomethyl-p-tolylsulfon als eine teilchenförmige Dispersion in einem flüssigen Lösungsmittel vorhanden ist, das aus Wasser oder einer Mischung von Wasser und mit Wasser mischbarem Hilfslösungsmittel besteht.

**2.** Zusammensetzung nach Anspruch 1, zusätzliche Tenside umfassend.

**3.** Zusammensetzung nach Anspruch 1, wobei mindestens eine 30%ige retinierte Aktivität erzielt wird, wenn das Diiodomethyl-p-tolylsulfon als eine teilchenförmige Dispersion in einem flüssigen Lösungsmittel vorhanden ist.

**4.** Zusammensetzung nach Anspruch 1, wobei mehr als 70 Gew.-% des Diiodomethyl-p-tolylsulfons in Form einer

teilchenförmigen Dispersion in einem flüssigen Lösungsmittel verbleiben.

5. Zusammensetzung nach Anspruch 1, wobei Diiodomethyl-p-tolylsulfon eine Teilchengröße mit einem $D_{50}$ von weniger als 30 Mikrometern, vorzugsweise mit einem $D_{50}$ von weniger als 15 Mikrometern, bevorzugter mit einem $D_{50}$ von weniger als 7,5 Mikrometern aufweist.

6. Zusammensetzung nach Anspruch 1, wobei Diiodomethyl-p-tolylsulfon basierend auf dem Gesamtgewicht der Zusammensetzung zu 0,001 Gew.% bis 10 Gew.-%, vorzugsweise zu 0,01 Gew.-% bis 5 Gew.-%, bevorzugter zu 0,1 Gew.-% bis 3 Gew.-% vorhanden ist.

7. Zusammensetzung nach Anspruch 1, wobei der pH-Wert der Zusammensetzung 2 bis 11, vorzugsweise 4 bis 9, bevorzugter 6 bis 8 beträgt.

8. Zusammensetzung nach Anspruch 1, wobei die Zusammensetzung ferner ein Dispergierhilfsmittel umfasst.

9. Zusammensetzung nach Anspruch 1, wobei die Zusammensetzung ferner ein Suspendiermittel umfasst.

10. Zusammensetzung nach einem der Ansprüche 1 bis 9, das kationische Polymer, basierend auf dem Gesamtgewicht der Zusammensetzung, zu 0,1 Gew.-% bis 1,0 Gew.-% umfassend.

11. Zusammensetzung nach einem der Ansprüche 1 bis 10, ferner ein nichtionisches Polymer umfassend, das ausgewählt ist aus Polyalkylenglycolen mit einem Molekulargewicht von mehr als 1000.

12. Zusammensetzung nach einem der Ansprüche 1 bis 11, ferner, basierend auf dem Gesamtgewicht der Zusammensetzung, zu 0,001 Gew.-% bis 10 Gew.-% ein unlösliches Silikon-Konditioniermittel umfassend.

13. Verwendung einer Zusammensetzung nach einem der Ansprüche 1 bis 12 zur Herstellung eines topisch aufgetragenen Medikaments für die Behandlung oder Verhütung von mikrobiellen Infektionen der Haut oder Kopfhaut.

**Revendications**

1. Composition comprenant :

   De 12 % en poids à 22 % en poids, basé sur le poids total de la composition, d'agent tensioactif détersif anionique ;
   De 0,05 % en poids à 3 % en poids, basé sur le poids total de la composition, d'un polymère cationique ; et
   une quantité efficace de di-iodométhyl-p-tolylsulfone, le di-iodométhyl-p-tolylsulfone étant présent en tant que dispersion particulaire dans un solvant liquide constitué d'eau ou d'un mélange d'eau et d'un cosolvant miscible dans l'eau.

2. Composition selon la revendication 1, comprenant des agents tensioactifs supplémentaires.

3. Composition selon la revendication 1, dans laquelle au moins 30 % de l'activité conservée est obtenue lorsque le di-iodométhyl-p-tolylsulfone est présent en tant que dispersion particulaire dans un solvant liquide.

4. Composition selon la revendication 1, dans laquelle plus de 70 % en poids du di-iodométhyl-p-tolylsulfone demeure sous la forme d'une dispersion particulaire dans un solvant liquide.

5. Composition selon la revendication 1, dans laquelle le di-iodométhyl-p-tolylsulfone a une taille des particules avec un $D_{50}$ de moins de 30 microns, de préférence avec un $D_{50}$ de moins de 15 microns, plus préférablement avec un $D_{50}$ de moins de 7,5 microns.

6. Composition selon la revendication 1, dans laquelle le di-iodométhyl-p-tolylsulfone est présent de 0,001 % en poids à 10 % en poids, de préférence de 0,01 % en poids à 5 % en poids, plus préférablement de 0,1 % en poids à 3 % en poids basé sur le poids total de la composition.

7. Composition selon la revendication 1, dans laquelle le pH de la composition va de 2 à 11, de préférence de 4 à 9, plus préférablement de 6 à 8.

8. Composition selon la revendication 1, où la composition comprend en outre un auxiliaire de dispersion.

9. Composition selon la revendication 1, où la composition comprend en outre un agent de suspension.

10. Composition selon l'une quelconque des revendications 1 à 9, comprenant de 0,1 à 1,0 % en poids, basé sur le poids total de la composition, du polymère cationique.

11. Composition selon l'une quelconque des revendications 1 à 10, comprenant en outre un polymère non ionique choisi parmi les polyalkylène glycols ayant une masse moléculaire de plus de 1000.

12. Composition selon l'une quelconque des revendications 1 à 11, comprenant en outre de 0,001 % en poids à 10 % en poids, basé sur le poids total de la composition, d'un agent de conditionnement siliconé insoluble.

13. Utilisation d'une composition comme définie selon l'une quelconque des revendications 1 à 12, pour la fabrication d'un médicament appliqué localement pour le traitement ou la prévention d'une infection microbienne de la peau ou du cuir chevelu.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2003096545 A1
- KR 0141416 B, Chang-Duk Kim
- EP 0034877 A
- US 2486921 A
- US 2486922 A
- US 2396278 A
- US 3332880 A
- US 5104646 A, Bolich Jr.
- US 5106609 A, Bolich Jr.
- US 3929678 A
- US 2658072 A
- US 2438091 A
- US 2528378 A
- US 3962418 A
- US 3958581 A
- US 34584 A
- US 4152416 A
- US 2826551 A
- US 3964500 A
- US 4364837 A
- GB 849433 A
- US 5674478 A
- US 5750122 A
- US 4529586 A
- US 4507280 A
- US 4663158 A
- US 4197865 A
- US 4217914 A
- US 4381919 A
- US 4422853 A
- US 2694668 A
- US 3152046 A
- US 4089945 A
- US 4885107 A
- US 4741855 A
- US 5837661 A
- US 5665364 A


### Non-patent literature cited in the description


- McCutcheon's, Emulsifiers and Detergents. M. C. Publishing Co, 1989
- CTFA Cosmetic Ingredient Dictionary. The Cosmetic, Toiletry, and Fragrance Association, Inc, 1982
- Encyclopedia of Polymer Science and Engineering. John Wiley & Sons, Inc, 1989, vol. 15, 204-308
- **Noll ; Walter.** Chemistry and Technology of Silicones. Academic Press, 1968
- Silicon Compounds. Petrarch Systems, Inc, 1984
- **E. Gueho et al.** *Antoinie Leeuwenhoek,* 1996, 337-55